(19) Europäisches Patentamt / European Patent Office / Office européen des brevets

(11) **EP 3 785 028 B1**

(12) **EUROPEAN PATENT SPECIFICATION**

(45) Date of publication and mention
of the grant of the patent:
**07.08.2024 Bulletin 2024/32**

(21) Application number: **19720556.0**

(22) Date of filing: **26.04.2019**

(51) International Patent Classification (IPC):
***G01N 33/50*** *(2006.01)*

(52) Cooperative Patent Classification (CPC):
**G01N 33/5008**

(86) International application number:
**PCT/EP2019/060786**

(87) International publication number:
**WO 2019/207129 (31.10.2019 Gazette 2019/44)**

(54) **UV FILTER COMPOSITIONS AND METHODS OF PREPARATION AND USE THEREOF**

UV-FILTERZUSAMMENSETZUNGEN UND VERFAHREN ZU IHRER HERSTELLUNG UND IHRER VERWENDUNG

COMPOSITIONS DE FILTRE UV ET PROCÉDÉS DE PRÉPARATION ET D'UTILISATION CORRESPONDANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.04.2018 US 201862663374 P**

(43) Date of publication of application:
**03.03.2021 Bulletin 2021/09**

(73) Proprietor: **BASF SE**
**67056 Ludwigshafen am Rhein (DE)**

(72) Inventors:
• **ACKER, Stephanie**
  **79639 Grenzach-Wyhlen (DE)**
• **PAWLOWSKI, Sascha**
  **67056 Ludwigshafen (DE)**
• **HERZOG, Bernd**
  **79639 Grenzach-Wyhlen (DE)**

(74) Representative: **BASF IP Association**
**BASF SE**
**GBI-C006**
**67056 Ludwigshafen (DE)**

(56) References cited:
**US-A1- 2006 004 474    US-A1- 2011 246 397**
**US-A1- 2015 100 533**

• **KAISER D ET AL: "Ecotoxicological effect characterisation of widely used organic UV filters", ENVIRONMENTAL POLLUTION, BARKING, GB, vol. 163, 2 December 2011 (2011-12-02), pages 84 - 90, XP028454780, ISSN: 0269-7491, [retrieved on 20111217], DOI: 10.1016/J.ENVPOL.2011.12.014**
• **PABLO GAGO-FERRERO ET AL: "An overview of UV-absorbing compounds (organic UV filters) in aquatic biota", ANALYTICAL AND BIOANALYTICAL CHEMISTRY, SPRINGER, BERLIN, DE, vol. 404, no. 9, 6 June 2012 (2012-06-06), pages 2597 - 2610, XP035130339, ISSN: 1618-2650, DOI: 10.1007/S00216-012-6067-7**

**Description**

**FIELD OF THE INVENTION**

The present disclosure relates to ultraviolet filter compositions, and methods relating to their preparation and use.

**BACKGROUND OF THE INVENTION**

There is a growing interest for environmentally friendly pharmaceutical, cosmetic and sunscreen products. Many countries have different regulations governing environmental standards of such products and approved chemical components and compositions may also differ between countries. Therefore, companies that sell such products in the global marketplace, must be able to readily determine if their products meet the requirements of a particular country.

Various techniques have been developed to assess environmental issues that may arise when formulating compositions. For example, "a grading system" based on a supplier's environmental practices, categorizing ingredients to determine an environmental "footprint" of a product, a "grading system" based solely on the toxicity of individual components and an environmental impact analysis the includes a decision framework to reduce the potential environmental impact of a product. However, such techniques do not take into consideration all of the available experimental physical-chemical, ecotoxicological and environmental fate data available for a particular component when assessing the environmental hazard of a component or formulation.

The environmental impact of ultraviolet ("UV") filters (e.g., as used in sunscreens) has been increasingly examined due to the damaging effects that certain such filters have on the environment. There remains a need for evaluating the environmental friendliness of individual components, such as UV filters, and further determining the overall eco-friendliness of a composition containing these individual components.

**SUMMARY**

The invention is set out in the appended claims.

In accordance with various embodiments herein, described are methods for formulating compositions containing such individual components, such as UV filters. Also described are methods of use.

According to various embodiments, described herein is a method of formulating a composition as defined in claims 1 to 5.

Further described herein are various embodiments of a composition having at least one or at least two UV filters; and at least one other ingredient selected from a surfactant, super-fatting agent, oil, emulsifier, wax, consistency regulator, thickener, polymers, silicon, siloxane, stabilizer, biogenic active ingredient, natural or synthetic triglyceride, fatty acid, fatty alcohol, ester of fatty acid, swelling agent, further UV light-protective agent, antioxidant, hydrotropic agent, preservative, solubiliser, perfume oil, colorant, bacteria-inhibiting agent, adjuvant and mixtures thereof.

According to further embodiments, described herein is a method of using an overall environmental hazard score of each of a plurality of ultraviolet (UV) filters to formulate a composition comprising an ecotoxicological value of from 0 to 350 as defined in claims 6 to 9.

**DEFINITIONS**

The term "ultraviolet filter" or "UV filter" means any organic and/or inorganic compound containing a chromophore group responsible for absorption and / or reflection and scattering of electromagnetic radiation in the wavelength range between 280 and 400 nm or up to 450 nm (including blue light range). The chromophore group consists of a system of conjugated double bonds and typically contains at least one aromatic moiety.

**BRIEF DESCRIPTION OF THE DRAWINGS**

The above and other features of the present disclosure, their nature, and various advantages will become more apparent upon consideration of the following detailed description, taken together with the accompanying drawings, in which:

Figure 1 illustrates a method of determining an environmental profile of an individual component and an overall environmental score of the individual component in accordance with embodiments disclosed herein.
Figure 2 illustrates a method of formulating an environmentally friendly composition in accordance with embodiments disclosed herein.

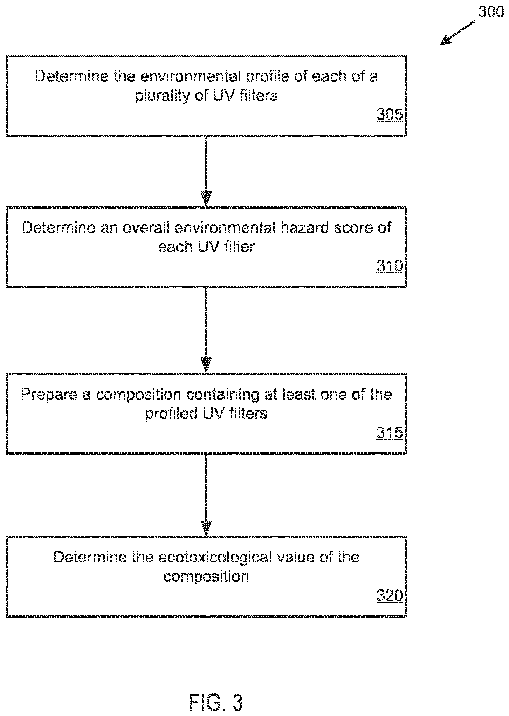

Figure 3 illustrates a method of formulating a composition in accordance with embodiments disclosed herein.

## DETAILED DESCRIPTION

Disclosed herein are methods of formulating compositions, which include determining an environmental profile of certain individual components, an overall environmental hazard score of each these components and an ecotoxicological value of a composition containing one or more of these components.

## Methods of Formulating a Composition

According to various embodiments, the methods of formulating a composition as described herein involve determining the environmental profile of a component (e.g., an UV filter) based on available experimental physical-chemical, ecotoxicological and environmental fate data. The component profiles are used to determine an overall environmental hazard score of the component. With this information, the components are ranked based on their environmental friendliness. One or more of the components is combined with one or more other ingredients to form a composition (i.e., actual or hypothetical) and the ecotoxicological value of the composition as a whole is determined.

### *Determining the Environmental Profile and Overall Ecotoxicological Value of Components*

According to various embodiments described herein, an environmental profile of an organic and/or inorganic component is determined based on available ecotoxicological and/or environmental fate data relating to various environmental chapters of the component. If such data is unavailable for one or more environmental chapter of the component, then basic core data about the component such as physical-chemical and environmental fate properties can be used to fill the data gap. If such core data is unavailable for one or more environmental chapter of the component, then a worst-case scenario is determined for the one or more environmental chapter of the component.

Once the environmental profile for a component has been determined, then an overall environmental hazard score may be determined for the component. Certain cut-off criteria, however, are also considered as will be described in more detail below.

### *Data Sources*

According to various embodiments described herein, methods of formulating a composition include determining the environmental profile of an individual component. To accomplish this, the following data sources were researched and/or used to obtain information for each individual component: 1) study reports (if available); 2) scientific based literature (if available); 3) information from governmental authorities (e.g., European Chemicals Agency - "ECHA" website) (if available); and 4) Quantitative Structure Activity Relationship ("QSAR") calculations (e.g., US EPA. 2018. Estimation Programs Interface Suite™ for Microsoft® Windows, v 4.11. United States Environmental Protection Agency, Washington, DC, USA.). As a first step, scientific literature was screened by available CAS numbers or substance names in combination with a selection of adequate key words. Relevant study reports were selected based on the CAS number and type of study. ECHA-Website information was screened according to the identification parameters of the individual substance (i.e. CAS or EC number). QSAR estimations were carried out using simplified molecular-input line-entry system (SMILES) codes derived from the available chemical structures or the CAS number itself. The data provided by the above sources falls within one or more of the following environmental chapters: 1) biodegradation, 2) bioaccumulation, 3) acute aquatic toxicity, 4) chronic aquatic toxicity, 5) chronic terrestrial toxicity, and 6) sediment toxicity.

In certain embodiments, where no information is available for one or more of the environmental chapters, an expert statement may be prepared to fill the "data gap." This expert statement is based on basic core ecotox and environmental fate data (e.g., acute aquatic toxicity data, screening data on biodegradation (OECD 301 and 302 series), adsorption potential, etc.), such as the available physical and chemical properties and the environmental fate properties of the individual component. A substance with a logarithmic octanol water partition (LogPow) higher than 4.5 is considered as bioaccumulative/very biaccumulative if adequate experimentally derived or valid estimated data were not available. In contrast, a substance with LogPow below 4.5 is considered as not bioaccumulative and not very bioaccumulative. In addition, a substance indicating poor biodegradability in the OECD 301 and 302 tests, but having the potential to adsorb to organic matter (as indicated by a high LogPow or LogKoc[octanol-carbon coefficient]), may be transferred to both soil and sediments. Therefore, additional data on sediment and terrestrial organisms are considered as required. In the absence of adequate data on soil and sediment organisms, a worst-case toxicity assessment is applied to these compartments. In case the screening criteria for adsorption indicate a low adsorption potential of the substance (as indicated by a low LogPow and or a low Koc-value), and/or the substance is known to be readily biodegradable, no indirect transfer to both soil and sediment is assumed and thus, no additional data for the soil and sediment compartment may be required are considered to be relevant. Due to the half-life of UV filters in both soil and sediments in the range of at least a few weeks (according to the Technical Guidance Document on Risk Assessment, European Commission, 2003, part II), chronic toxicity tests instead of acute toxicity tests are considered as adequate to address these compartments. Acute toxicity tests on both soil and sediment organisms were only considered as indicative but not as definitive for the assessment. For inorganic UV filters, the biodegradation criteria are considered as not applicable; however, due to the long-lasting existence in the environmental compartments (i.e. water, sediment and soil) and thus their long-lasting toxicity effects, once emitted into these, the biodegradation profile was rated as a worst case

In further embodiments, where basic core data is unavailable for one or more environmental chapter of a component, a worst-case assumption is made, which provides the highest reasonable scoring the for the environmental chapter for which data is missing. As will be described in more detail below, a relatively higher score indicates that a component has relatively poorer environmental characteristics.

*Reliability of Data*

According to various embodiments, data collected from study reports, scientific based literature and governmental authorities were assessed for reliability. In certain embodiments, such reliability was assessed using criteria set forth by Klimisch et al., 1997 (i.e., reliability 1 - reliable; reliability 2 - reliable with restrictions; reliability 3 - not reliable; reliability 4 - not assessable). Only data having a reliability 1 and 2 were used to determine the environmental profiles of the corresponding chemical component.

*Determining Environmental Chapters*

According to various embodiments, environmental chapters were determined by grouping relevant data into the following categories: 1) Environmental Fate (Biodegradation, Bioaccumulation); and 2) Ecotoxicity (Acute aquatic toxicity, Chronic aquatic toxicity, Terrestrial toxicity, Sediment toxicity). In order to provide a ranking of the individual component based on its environmental fate end ecotoxicological profile, criteria were defined for each environmental chapter and the available data within these chapters were ranked by a given number. Components with the best environmental behavior in a specific environmental chapter are indicated with the lowest number (i.e., 0.25), whereas substances having the most negative impact within the environmental chapter are rated with the highest number.

To provide equal value to each of the environmental chapters, each chapter starts with 0.25 as the lowest value; however; for both environmental fate related chapters (i.e., biodegradation and bioaccumulation), the highest achievable (worst case) value is 1, whereas for the ecotoxicological related chapters, in theory no upper (highest) threshold value can be given, as toxicity may be given even beyond this value. In such instances, the value will increase stepwise by 0.25 in order to take into account substances with a specific higher toxicity. Nevertheless, the upper value of 1 may be sufficient to assess the ecotoxicological effects of most components. For ecotoxicological chapters, the lowest score was applied to all substances showing no effects up to the highest test concentration tested (as recommended by the corresponding Organisation for Economic Co-operation and Development - "OECD" test guideline) or up to the limit of solubility under test conditions.

The scores for the environmental chapters were assessed as follows:

1. Biodegradation (According to OECD criteria)

| | |
|---|---|
| Readily biodegradable/not persistent | Factor 0.25 |
| Biodegradable/ not persistent | Factor 0.5 |
| Partly biodegradable/potential persistent | Factor 0.75 |
| Poorly biodegradable/persistent | Factor 1 |

2. Bioaccumulation

| | | |
|---|---|---|
| Bioaccumulation factor (BCF) | < 500 | Factor 0.25 |
| Bioaccumulation factor (BCF) | >= 500 - < 2000 | Factor 0.5 |
| Bioaccumulation factor (BCF) | >= 200 - < 5000 | Factor 0.75 |
| Bioaccumulation factor (BCF) | > 5000 | Factor 1.0 |

3. Acute aquatic toxicity

| | |
|---|---|
| EC50 > 100 mg/L or > WL | Factor 0.25 |
| EC50 ≤ 100 ≥ 10 mg/L | Factor 0.5 |
| EC50 < 10 ≥ 1 mg/L | Factor 0.75 |

(continued)

| EC50 < 1 mg/L | Factor 1 |
| *EC50 10 times lower as previously* | *+ 0.25* |

#### 4. Chronic aquatic toxicity

| NOEC/EC10 ≥ 10 mg/L | Factor 0.25 |
| NOEC/EC10 < 10 ≥ 1 mg/L | Factor 0.5 |
| NOEC/EC10 < 1 ≥ 0.1 mg/L | Factor 0.75 |
| NOEC/EC10 < 0.1 ≥ 0.01 mg/L | Factor 1 |
| *NOEC/EC10 10 times lower* | *+ 0.25* |

#### 5. Chronic terrestrial toxicity

| NOEC/EC10 ≥ 1000 mg/kg | Factor 0.25 |
| NOEC/EC10 < 1000 ≥ 100 mg/kg | Factor 0.5 |
| NOEC/EC10 < 100 ≥ 10 mg/kg | Factor 0.75 |
| NOEC/EC10 < 10 ≥ 1 mg/kg | Factor 1 |
| *NOEC/EC10 10 times lower* | *+ 0.25* |

#### 6. Sediment toxicity

| NOEC/EC10 ≥ 1000 mg/kg | Factor 0.25 |
| NOEC/EC10 < 1000 ≥ 100 mg/kg | Factor 0.5 |
| NOEC/EC10 < 100 ≥ 10 mg/kg | Factor 0.75 |
| NOEC/EC10 < 10 ≥ 1 mg/kg | Factor 1 |
| *NOEC/EC10 10 times lower* | *+ 0.25* |

It should be noted that each environmental chapter was given the same level of concern in the environmental profile of the individual component. For example, aquatic toxicity is not of higher or lower concern than endocrine or bioaccumulation.

*Cut-Off Criteria*

According to various embodiments, cut-off criteria may be used to exclude potentially harmful components from being formulated into compositions. For example, components under suspicion of endocrine disruption ("ED") and even proven to be endocrine active for the environment as well as substances considered or confirmed to be persistent/bioaccumulative/toxic ("PBT") or very persistent/very bioaccumulative ("vPvB") may provide a serious threat to the environment due to their wide dispersive use, despite other more favorable properties within their environmental profile. In certain embodiments, criteria set forth in the ECHA PBT guidance document, Chapter R11 PBT Assessment (available at: https://echa.europa.eu/de/guidance-documents/guidance-on-information-requirements-and-chemical-safety-assessment, last accessed Mar. 12, 2018) may also be considered. According to certain embodiments, endocrine activity, ED, PBT and/or vPvB may be used as cut-off criteria to exclude a particular component from use. Therefore, components falling into at least one of these categories (endocrine active, ED, PBT, vPvB), may be considered as not suitable for further assessment of ecofriendly formulations and thus, may be excluded from further assessment.

*Determining Overall Environmental Hazard Score*

The environmental profile of a component is used to determine the overall environmental hazard score for the component. As discussed above, there are six (6) environmental chapters each having a score ranging from 0.25 to typically 1.0 or 1.25. To determine the overall environmental hazard score of an individual component, the score in each of the environmental chapters are added together. Therefore, the minimum possible overall environmental hazard score is 1.5 and the maximum overall environmental hazard score is 7.0. An individual component has an overall environmental hazard score somewhere within this range.

*Ranking UV Filters*

Taking into account the cut-off criteria and the overall environmental hazard scores, individual components suitable for use in environmentally friendly compositions can be ranked. For example, if the individual components are all UV filters, then filters with a low overall environmental hazard scoring number have a better environmental profile as compared to filters with higher scoring numbers.

### *Ecotoxicological Ranking of Compositions*

According to various embodiments described herein, once the overall environmental hazard score of one or more individual components is known, then such components can be incorporated into a composition. For example, a mixture of UV filters may be formulated into a sunscreen, which can then be evaluated for its overall eco-friendliness. According to certain embodiments, the "eco-friendliness" of the sunscreen can be determined. In order to take the concentration of a UV filter into account, its overall environmental hazard score is multiplied by its concentration (in wt%) in the sunscreen.

As described above, the overall environmental hazard score for an individual component is in the range of 1.5 - 7.0. Thus, a sunscreen formulation containing, for example, UV filters at 1 wt% concentration would exhibit an ecotoxicological ranking value between 1.5 and 7.0, and a sunscreen formulation containing for instance 25 wt% of UV filters would lie in a range between 37.5 and 175. This range of minimal and maximal ecotoxicity is calculated according to the overall concentration in weight percent of the given UV filter composition.

The actual ecotoxicological ranking value of the composition will be somewhere in between this range. To obtain this quantity, the ecotoxicological ranking value of each individual UV filter in the composition is multiplied with the concentration of the respective filter, and all these products are summed up resulting in a value designated as (ecorank)i. The theoretical maximum ranking of the composition i is named (max-ecorank)i. Relating (ecorank)$_i$ to (max-ecorank)$_i$ yields the relative ecotoxicity of the given composition. In order to turn that relative ecotoxicity into a scale of eco-friendliness, the following transformation is made:

$$(\%eco - friendlyness)_i = \left\{ 1 - \frac{(ecorank)_i}{(max - ecorank)_i} \right\} \cdot 100$$

The higher the percentage, the friendlier the sunscreen formulation for the environment. This calculation method enables a comparison of the environmental impact of different sunscreen formulations and optimizes the formulation to be more environmentally compliant. In order to take into consideration the efficiency of a given UV filter composition in terms of achieving a certain SPF value and UVA-PF value, the value of the eco-friendliness is multiplied by the filter-efficiency, which is the SPF and UVA-PF divided by the total filter concentration in %. The SPF value and UVA-PF value are obtained by calculation using the sunscreen simulator or by in vivo or in vitro studies or used as claimed. In some embodiments, an EcoSun pass value can be represented as:

$$EcoSun\ Pass\ value = a \frac{SPF\ + UVA - PF}{[UV\ filters]}$$

wherein "a" corresponds to the ecotoxicological evaluation of the used filter system; "SPF" corresponds to the numerical value for the SPF obtained from a sunscreen simulator or obtained from in vivo or in vitro studies or using the claimed SPF, and "UVA-PF" is the numerical value for the UVA protection factor from a sunscreen simulator or from in vivo or in vitro studies or using the claimed UVA-PF. Sunscreen simulators calculate the SPF and the UVA-PF based on a database with quantitative UV spectra of the relevant UV filters, a mathematical description of the irregularity profile of the sunscreen film on the skin, and the consideration of changes in UV filter concentration due to photoinstabilities as described by B. Herzog and U. Osterwalder in "Simulation of sunscreen performance" (Pure Appl. Chem. 2015; 87 (9-10): 937-951). Further, "[UV filters]" refers to the UV filter concentration in weight-%. The above equation also takes account of the UVA protection provided by a given UV filter composition, and is therefore preferred for the calculation of the EcoSun pass value, i.e. the ecotoxicological value. Following the above equation, the ecotoxicological values are in the range of from 0 to 350 or preferably from 1.5 to 300.

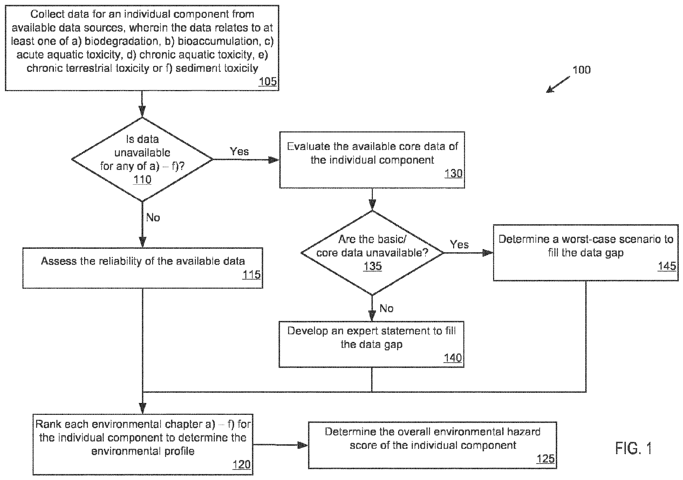

The methods of formulating compositions now will be described in accordance with FIGs 1-2. According to various embodiments described herein, as shown in FIG. 1, a method of formulating a composition (e.g., a sunscreen) 100 includes, at 105, collecting data for one or more individual components from available data sources, wherein the data relates to the environmental fate and ecotoxicological properties of the UV filter, in particular, the data relates to 1) biodegradation, 2) bioaccumulation, 3) acute aquatic toxicity, 4) chronic aquatic toxicity, 4) chronic terrestrial toxicity, and 6) sediment toxicity. If, at 110, such data is available for an environmental chapter, then, at 115, the reliability of the available data for that environmental chapter is assessed. Assessing reliability of the experimental data includes assigning a value of 1.5 to 7 to the experimental data, wherein only data with a reliability of about 1.5 to about 2, or 1.5 or 2 are used for the environmental profile.

If, however, at 110, data is unavailable for the six (6) environmental chapters, then, at 130, basic core data for the one or more individual components is evaluated. If, at 135, the basic core data is available for an individual component, then, at 140, an expert statement is prepared to fill the "data gap" for the particular environmental chapter. If, however, at 135, basic core data for the individual component is unavailable, then, at 145, a worst case score is used for the one or more environmental chapter for which the data is missing in order to fill the "data gap." In other words, the maximum value was applied for the environmental chapter. Research substances with only a minimum data set available may be at the lower end of the individual component ranking, similar to substances with a bad ecotox/efate profile. Fully tested substances having no known negative impact on the environment are ranked with an overall low number.

Once each environmental chapter for an individual component can be scored, then, at 120, the environmental profile for that particular component is determined. As indicated above, determining the environmental profile of an individual component is based on at least one data source selected from experimental data, ecotoxicological data, environmental fate data, study reports, scientific-based literature, information from government authorities and qualitative structure activity relationship models. In certain embodiments, determining the environmental profile comprises evaluating experimental data for a UV filter and if no experimental data is available, then calculating or estimating data to substitute for the experimental data. If calculating or estimating data to substitute for the experimental data is impossible, then a worst-case score is provided for the environmental chapter.

The environmental profile of an individual component includes a rating ranging from 0.25 to 1 for at least one of biodegradation or bioaccumulation, wherein a rating of 0.25 represents the best environmental behavior and a rating of 1.0 represents the poorest environmental behavior. The environmental profile also includes a rating of at least 0.25 for at least one of acute aquatic toxicity, chronic aquatic toxicity, chronic terrestrial toxicity or sediment toxicity, wherein a rating of 0.25 represents the best environmental behavior.

At 125, the environmental profile for an individual component is used to determine the overall environmental hazard score of the component. One or more of the individual components can be incorporated into a composition, such as a sunscreen. In certain embodiments, determining the ecotoxicological evaluation of the used filter system "a" of the composition includes multiplying the overall environmental hazard score for each UV filter by its respective concentration (by weight) in the composition and adding together the resulting values for each UV filter.

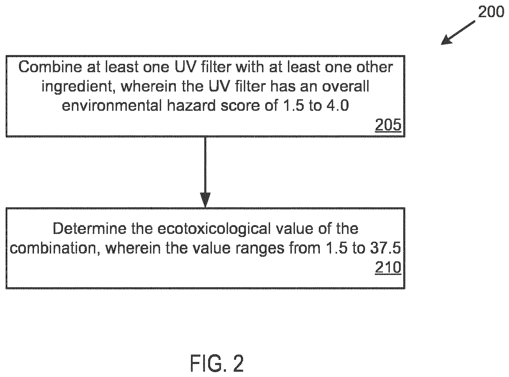

According to various embodiments of the disclosure, FIG. 2 illustrates a method of formulating a composition 200. At 205, at least one UV filter is combined with a at least one other ingredient (described in further detail below). According to various embodiments, the at least one other ingredient may form a base of a sunscreen composition. The UV filter has an overall environmental hazard score of 1.5 to 7.0. The overall environmental hazard score is a sum of ratings for six environmental chapters selected from a group consisting of biodegradation, bioaccumulation, acute aquatic toxicity, chronic aquatic toxicity, chronic terrestrial toxicity, sediment toxicity and combinations thereof.

At 210, the ecotoxicological evaluation of the used filter system "a"of the combined composition is determined. Determining the ecotoxicological evaluation of the used filter system "a" of the composition includes multiplying the overall environmental hazard score for each UV filter by its respective concentration (by weight) in the composition and adding together the resulting values for each UV filter. It corresponds to the factor a in the EcoSun Pass equation.

**Compositions**

**UV Filters**

Compositions formed in accordance with embodiments described herein can include one or more UV filters. Suitable UV filters that may be incorporated into a pharmaceutical, cosmetic or sunscreen formulation as described herein include, but are not limited to, ethylhexyl triazone (EHT), dimethyl para-aminobenzoate (ED PABA), PEG-25 PABA, ethylhexyl salicylate (EHS), ethylhexyl methoxy cinnamate (EHMC), iso-amyl methoxy cinnamate (IMC), octocrylene (OCR), phenyl benzimidazole sulfonic acid (PBSA), 4-methyl benzylidene camphor (4-MBC), ethylhexyl triazone (EHT), butyl methoxy dibenzoylmethane (BMDBM), terephthalidine dicamphor sulfonic acid (TDSA), zinc oxide in water, zinc oxide in oil, titanium oxide in water, titanium dioxide in oil, drometrizole trisiloxane (DTS), methylene bis-benzotriazolyl tetramethylbutylphenol (MBBT), bis-ethylhexyloxyphenol methoxyphenyl triazine (BEMT), benzophenone-3 (B3), benzophenone-4 (B4), menthyl anthranilate (MA), diethylhexyl butamido triazone (DBT), benzylidene malonate polysiloxane (BMP), disodium phenyl dibenzimidazole tetrasulfonate (DPDT), homomentyl salicylate (HMS), diethylamino hydroxybenzoyl hexyl benzoate (DHHB), tris bipenyl triazine (TBT), piperazine bi aminobenzophenone, bis-ethylhexyloxyphenol methoxyphenyl triazine (BEMT) in water.

*Other Ingredients*

Compositions in accordance with embodiments described herein may include, in addition to one or more UV filters, at least one other ingredient. Such compositions may be formulated for pharmaceuticals, cosmetics and/or sunscreen applications. The compositions may be in the form of, creams, gels, lotions, alcoholic and aqueous/alcoholic solutions, emulsions, wax/fat compositions, stick preparations, powders or ointments. In addition to the above-mentioned zinc oxide particles, the preparations may contain further cosmetically acceptable adjuvants.

The formulations can include one or more ingredients that are suitable for skin exposure. Such ingredients include, but are not limited to, surfactants, super-fatting agents, oils, emulsifiers, consistency regulators, thickeners, polymers, stabilizers, biogenic active ingredients, swelling agents, further UV light-protective agents, antioxidants, hydrotropic agents, preservatives, solubilisers, perfume oils, colorants, bacteria-inhibiting agents and mixtures thereof.

In certain embodiments, the formulation is a sunscreen. For example, a sunscreen containing water and oil such as W/O, O/W, O/W/O and W/O/W emulsions, or microemulsions. These emulsions may contain, for example, one or more UV filter at a concentration of 0.1 to 30% by weight, or 0.1 to 15% by weight, or 0.5 to 10% by weight, based on the total weight of the composition.

*Fatty Alcohols*

Guerbet alcohols based on fatty alcohols having from 6 to 18, preferably from 8 to 10 carbon atoms including cetyl alcohol, stearyl alcohol, cetearyl alcohol, oleyl alcohol, octyldodecanol, benzoate of C12-C15 alcohols, acetylated lanolin alcohol, hexyldecanol, etc.

*Esters of Fatty Acids*

Esters of linear $C_6$-$C_{24}$ fatty acids with linear $C_3$-$C_{24}$ alcohols, esters of branched $C_6$-$C_{13}$carboxyl acids with linear $C_6$-$C_{24}$ fatty alcohols, esters of linear $C_6$-$C_{24}$ fatty acids with branched alcohols, especially 2-ethylhexanol, esters of hydroxycarboxylic acids with linear or branched $C_6$-$C_{22}$ fatty alcohols, especially dioctyl malates, esters of linear and/or branched fatty acids with polyhydric alcohols (for example propylene glycol, dimer diol or trimer triol) and/or Guerbet alcohols, for example caproic acid, caprylic acid, 2-ethylhexanoic acid, capric acid, lauric acid, isotridecanoic acid, myristic acid, palmitic acid, palmitoleic acid, stearic acid, isostearic acid, oleic acid, elaidic acid, petroselinic acid, linoleic acid, linolenic acid, elaeostearic acid, arachidic acid, gadoleic acid, behenic acid and erucic acid and technical-grade mixtures thereof (obtained, for example, in the pressure removal of natural fats and oils, in the reduction of aldehydes from Roelen's oxosynthesis or in the dimerisation of unsaturated fatty acids) with alcohols, for example, isopropyl alcohol, caproic alcohol, capryl alcohol, 2-ethylhexyl alcohol, capric alcohol, lauryl alcohol, isotridecyl alcohol, myristyl alcohol, cetyl alcohol, palmoleyl alcohol, stearyl alcohol, isostearyl alcohol, oleyl alcohol, elaidyl alcohol, petroselinyl alcohol, linoyl alcohol, linolenyl alcohol, elaeostearyl alcohol, arachidyl alcohol, gadoleyl alcohol, behenyl alcohol, erucyl alcohol and brassidyl alcohol and technical-grade mixtures thereof (obtained, for example, in the high-pressure hydrogenation of technical-grade methyl esters based on fats and oils or aldehydes from Roelen's oxosynthesis and as monomer fractions in the dimerisation of unsaturated fatty alcohols).

Examples of such ester oils are isopropylmyristate, isopropylpalmitate, ethylhexyl palmitate, isopropylstearate, isopropyl isostearate, isopropyloleate, n-butylstearate, n-hexyllaurate, n-decyloleate, isooctylstearate, iso-nonylstearate, isononyl isononanoate, cetearyl isononanoate, 2-ethylhexylpalmitate, 2-hexyllaurate, hexyldexyl laurate, 2-hexyldecyl stearate, 2-octyldodecylpalmitate, oleyloleate, oleylerucate, erucyloleate, erucylerucate, cetearyl octanoate, cetyl palmitate, cetyl stearate, decyl oleate, cetyl oleate, cetyl behenate, cetyl acetate, myristyl myristate, myristyl behenate, myristyl oleate, myristyl stearate, myristyl palmitate, myristyl lactate, propylene glycol dicaprylate/caprate, propylheptyl caprylate, dicaprylyl carbonate, caprylyl-caprylate/caprate, coco-caprylate/caprate, coco caprylate, stearyl heptanoate, diisostearyl malate, octyl hydroxystearate, etc.

*Other Adjuvants*

Diethylhexyl 2,6-naphthalate, di-n-butyl adipate, di(2-ethylhexyl)-adipate, di(2-ethyl hexyl)-succinate and diisotridecyl acelaat, and also diol esters, such as ethylene glycol dioleate, ethylene glycol diisotridecanoate, propylene glycol di(2-ethylhexanoate), propylene glycol diisostearate, propylene glycol dipelargonate, butanediol diisostearate and neopentyl glycol dicaprylate. Esters of $C_6$-$C_{24}$ fatty alcohols and/or Guerbet alcohols with aromatic carboxylic acids, saturated and/or unsaturated, especially benzoic acid, esters of $C_2$-$C_{12}$ dicarboxylic acids with linear or branched alcohols having from 1 to 22 carbon atoms or polyols having from 2 to 10 carbon atoms and from 2 to 6 hydroxy groups.

*Natural or Synthetic Triglycerides Including Glyceryl Esters and Derivatives*

Di- or tri-glycerides, based on $C_6$-$C_{18}$ fatty acids, modified by reaction with other alcohols (caprylic/capric triglyceride, cocoglycerides, wheat germ glycerides, etc.). Fatty acid esters of polyglycerin (polyglyceryl-n such as polyglyceryl-4 caprate, polyglyceryl-2 isostearate, etc. or castor oil, hydrogenated vegetable oil, sweet almond oil, wheat germ oil, sesame oil, hydrogenated cottonseed oil, coconut oil, avocado oil, corn oil, hydrogenated castor oil, shea butter, cocoa butter, soybean oil, mink oil, sunflower oil, safflower oil, macadamia nut oil, olive oil, hydrogenated tallow, apricot kernel oil, hazelnut oil, borago oil, etc.

Waxes including esters of long-chain acids and alcohols as well as compounds having wax-like properties, e.g., carnauba wax, beeswax (white or yellow), lanolin wax, candellila wax, ozokerite, japan wax, paraffin wax, microcrystalline wax, ceresin, cetearyl esters wax, synthetic beeswax, etc. Also, hydrophilic waxes as Cetearyl Alcohol or partial glycerides.

*Pearlescent Waxes*

Alkylene glycol esters, especially ethylene glycol distearate, fatty acid alkanolamides, especially coco fatty acid diethanolamide, partial glycerides, especially stearic acid monoglyceride, esters of polyvalent, unsubstituted or hydroxy-substituted carboxylic acids with fatty alcohols having from 6 to 22 carbon atoms, especially long-chained esters of tartaric acid, fatty substances, for example fatty alcohols, fatty ketones, fatty aldehydes, fatty ethers and fatty carbonates, which in total have at least 24 carbon atoms, especially lauryl and distearyl ether, fatty acids, such as stearic acid, hydroxystearic acid or behenic acid, ring-opening products of olefin epoxides having from 12 to 22 carbon atoms with fatty alcohols having from 12 to 22 carbon atoms and/or polyols having from 2 to 15 carbon atoms and from 2 to 10 hydroxy groups, and mixtures thereof.

*Hydrocarbon Oils*

Mineral oil (light or heavy), petrolatum (yellow or white), microcrystalline wax, paraffinic and isoparaffinic compounds, hydrogenated isoparaffinic molecules as polydecenes and polybutene, hydrogenated polyisobutene, squalane, isohexadecane, isododecane and others from plant and animal kingdom.

*Silicones or Siloxanes (Organosubstituted Polysiloxanes)*

Dimethylpolysiloxanes, methylphenylpolysiloxanes, cyclic silicones, and also amino-, fatty acid-, alcohol-, polyether-, epoxy-, fluorine-, glycoside- and/or alkyl-modified silicone compounds, which at room temperature may be in either liquid or resinous form. Linear polysiloxanes, dimethicone (Dow Corning 200 fluid, Rhodia Mirasil DM), dimethiconol, cyclic silicone fluids, cyclopentasiloxanes volatiles (Dow Corning 345 fluid), phenyltrimethicone (Dow Corning 556 fluid). Also suitable are simethicones, which are mixtures of dimethicones having an average chain length of from 200 to 300 dimethylsiloxane units with hydrogenated silicates. A detailed survey by Todd et al. of suitable volatile silicones may in addition be found in Cosm. Toil. 91, 27 (1976).

*Emulsifiers*

Any conventionally usable emulsifier can be used for the compositions. Emulsifier systems may comprise for example: carboxylic acids and their salts: alcalin soap of sodium, potassium and ammonium, metallic soap of calcium or magnesium, organic basis soap such as Lauric, palmitic, stearic and oleic acid etc.... Alkyl phosphates or phosphoric acid esters, acid phosphate, diethanolamine phosphate, potassium cetyl phosphate. Ethoxylated carboxylic acids or polyethyleneglycol esters, PEG-n acylates. Linear fatty alcohols having from 8 to 22 carbon atoms, branched from 2 to 30 mol of ethylene oxide and/or from 0 to 5 mol propylene oxide with fatty acids having from 12 to 22 carbon atoms and with alkylphenols having from 8 to 15 carbon atoms in the alkyl group. Fatty alcohol polyglycolether such as laureth-n, ceteareth-n, steareth-n, oleth-n. Fatty acid polyglycolether such as PEG-n stearate, PEG-n oleate, PEG-n cocoate. Monoglycerides and polyol esters. C12-C22 fatty acid mono- and di-esters of addition products of from 1 to 30 mol of ethylene oxide with polyols. Fatty acid and polyglycerol ester such as monostearate glycerol, diisostearoyl polyglyceryl-3-diisostearates, polyglyceryl-3-diisostearates, triglyceryl diisostearates, polyglyceryl-2-sesquiisostearates, polyglyceryl-2 dipolyhydroxystearate, or polyglyceryl dimerates. Mixtures of compounds from a plurality of those substance classes are also suitable. Fatty acid polyglycolesters such as monostearate diethylene glycol, fatty acid and polyethylene glycol esters, fatty acid and saccharose esters such as sucro esters (sucrose polystearate, etc), glycerol and saccharose esters such as sucro glycerides. Sorbitol and sorbitan, sorbitan mono- and di-esters of saturated and unsaturated fatty acids having from 6 to 22 carbon atoms and ethylene oxide addition products. Polysorbate-n series, sorbitan esters such as sesquiisostearate, sorbitan, PEG-(6)-isostearate sorbitan, PEG-(10)-sorbitan laurate, PEG-17-dioleate sorbitan. Glucose derivatives, C8-C22 alkyl-mono and oligo-glycosides and ethoxylated analogues with glucose being preferred as the sugar component. O/W emulsifiers such as methyl gluceth-20 sesquistearate, sorbitan stearate/sucrose cocoate, methyl glucose sesquistearate, cetearyl alcohol/cetearyl glucoside, lauryl glucoside. W/O emulsifiers such as methyl glucose dioleate/methyl glucose isostearate. Sulfates and sulfonated derivatives, dialkylsulfosuccinates (disodium cetearyl sulfosuccinate, etc), dioctyl succinate, alkyl lauryl sulfonate, linear sulfonated parafins, sulfonated tetraproplyne sulfonate, sodium lauryl sulfates, amonium and ethanolamine lauryl sulfates, lauyl ether sulfates, sodium laureth sulfates, sulfosuccinates, aceyl isothionates, alkanolamide sulfates, taurines, methyl taurines, imidazole sulfates. Polysiloxane/polyalkyl/polyether copolymers and derivatives, dimethicone, copolyols, silicone polyethylene oxide copolymer, silicone glycol copolymer. Propoxylated or POE-n ethers (Meroxapols), Polaxamers or poly(oxyethylene)m-block-poly(oxypropylene)n-block(oxyethylene). Zwitterionic surfactants that carry at least one quaternary ammonium group and at least one carboxylate and/or sulfonate group in the molecule. Zwitterionic surfactants that are especially suitable are betaines, such as N-alkyl-N,N-dimethylammonium glycinates, cocoalkyldimethylammonium glycinate, N-acylaminopropyl-N,N-dimethylammonium glycinates, cocoacylaminopropyldimethylammonium glycinate and 2-alkyl-3-carboxymethyl-3-hydroxyethylimidazolines each having from 8 to 18 carbon atoms in the alkyl or acyl group and also cocoacylaminoethylhydroxyethylcarboxymethylglycinate, N-alkyl betaine, N-alkylaminobetaines. Alkylimidazolines, alkylopeptides, lipoaminoacides, self emulsifying bases and the compounds as described in K. F. DePolo, A short textbook of cosmetology, Chapter 8, Table 8-7, p 250-251.

Nonionic bases such as PEG-6 beeswax (and) PEG-6 stearate (and) polyglyceryl-2-isostearate, glyceryl stearate (and) PEG-100 stearate, PEG-5 glyceryl stearate, sorbitan oleate (and) polyglyceryl-3 ricinoleate, sorbitan stearate and sucrose cocoate, glyceryl stearate and laureth-23, cetearyl alcohol and ceteth-20, cetearyl alcohol and colysorbate 60 and PEG-150 and stearate-20, cetearyl alcohol and cetearyl polyglucoside, cetearyl alcohol and ceteareth-20, cetearyl alcohol and PEG-40 castor oil, cetearyl alcohol and PEG-40 castor oil and sodium cetearyl sulfate, stearyl alcohol and steareth-7 and steareth-10, cetearyl alcohol and szeareth-7 and steareth-10, glyceryl stearate and PEG-75 stearate, propylene glycol ceteth-3 acetate, propylene glycol isoceth-3 acetate, cetearyl alcohol and ceteth-12 and oleth-12, PEG-6 stearate and PEG-32 stearate, PEG-6 stearate and ceteth-20 and steareth-20, PEG-6 stearate and ceteth-20 and glyceryl stearate and steareth-20, glyceryl stearate and ceteareth-20.

Anionic alkaline bases such as PEG-2 stearate SE, glyceryl stearate SE, propylene glycol stearate. Anionic acid bases such as cetearyl Alcohol and Sodium cetearyl sulfate, cetearyl alcohol and sodium lauryl sulfate, trilaneth-4 phosphate and glycol stearate and PEG-2 stearate, glyceryl stearate and sodium lauryl Sulfate, sodium stearoyl glutamate. Cationic acid bases such as cetearyl alcohol and cetrimonium bromide.

The emulsifiers may be used in an amount of, for example, from 1 to 30% by weight, especially from 4 to 20% by weight and preferably from 5 to 10% by weight, based on the total weight of the composition.

When formulated in O/W emulsions, the preferably amount of such emulsifier system could represent 5% to 20% of the oil phase.

*Adjuvants and Additives*

Compositions described herein, for example, creams, gels, lotions, alcoholic and aqueous/alcoholic solutions, emulsions, wax/fat compositions, stick preparations, powders or ointments, may in addition contain, further adjuvants and additives such as, mild surfactants, super-fatting agents, consistency regulators, thickeners, polymers, stabilisers, biogenic active ingredients, swelling agents, further UV light-protective factors, antioxidants, hydrotropic agents, preservatives, self-tanning agents, solubilisers, perfume oils, colourants, bacteria-inhibiting agents and the like.

*Super-Fatting Agents*

Substances suitable for use as super-fatting agents are, for example, lanolin and lecithin and also polyethoxylated or acrylated lanolin and lecithin derivatives, polyol fatty acid esters, monoglycerides and fatty acid alkanolamides, the latter simultaneously acting as foam stabilisers.

*Surfactants*

Examples of suitable mild surfactants, that is to say surfactants especially well tolerated by the skin, include fatty alcohol polyglycol ether sulfates, monoglyceride sulfates, mono- and/or di-alkyl sulfosuccinates, fatty acid isethionates, fatty acid sarcosinates, fatty acid taurides, fatty acid glutamates, .alpha.-olefin sulfonates, ethercarboxylic acids, alkyl oligoglucosides, fatty acid glucamides, alkylamidobetaines and/or protein fatty acid condensation products, the latter preferably being based on wheat proteins.

*Consistency Regulators/Thickeners and Rheology Modifiers*

Silicium dioxide, magnesium silicates, aluminum silicates, polysaccharides or derivatives thereof for example hyaluronic acid, xanthan gum, guar-guar, agar-agar, alginates, carraghenan, gellan, pectines, or modified cellulose such as hydroxycellulose, hydroxypropylmethylcellulose. In addition polyacrylates or homopolymer of reticulated acrylic acids and polyacrylamides, carbomer (RHEOCARE C Plus, CARBOPOL types 980, 981, 1382, ETD 2001, ETD2020, ULTREZ 10) or SALCARE range such as SALCARE SC80 (steareth-10 allyl ether/acrylates copolymer), Salcare SC81 (acrylates copolymer), TINOVIS ADE (sodium acrylates copolymer (and) PPG-1 trideceth-6), COSMEDIA ACE (sodium polyacrylates (and) Polyglyceryl-3 Caprate), COSMEDIA SP (sodium polyacrylate), HISPAGEL 200 (glycerin (and) glyceryl acrylate), SEPIGEL 305 (polyacrylamide/laureth-7), SIMULGEL NS and SIMULGEL EG (hydroxyethyl acrylate/sodium acryloyldimethyl taurate copolymer), STABILEN 30 (acrylates/vinyl isodecanoate crosspolymer), PEMULEN TR-1 (acrylates/C10-30 alkyl acrylate crosspolymer), LUVIGEL EM (sodium acrylates copolymer), TINOVIS GTC (acrylates/beheneth-25 methacrylate copolymer), etc. In addition, cationic rheology modifiers such as Polyquaternium-37 under the trade name COSMEDIA ULTRAGEL 300 or COSMEDIA TRIPLE C.

*Polymers*

As anionic, zwitterionic, amphoteric and non-ionic polymers there come into consideration, for example, vinyl acetate/crotonic acid copolymers, vinylpyrrolidone/vinyl acrylate copolymers, vinyl acetate/butyl maleate/isobornyl acrylate copolymers, methyl vinyl ether/maleic anhydride copolymers and esters thereof, uncrosslinked polyacrylic acids and polyacrylic acids crosslinked with polyols, acrylamidopropyl-trimethylammonium chloride/acrylate copolymers, octyl acrylamide/methyl methacrylate-tert-butylaminoethyl methacrylate/2-hydroxypropyl methacrylate copolymers, polyvinylpyrrolidone, vinylpyrrolidone/vinyl acetate copolymers, vinylpyrrolidone/dimethylaminoethyl methacrylate/vinyl caprolactam terpolymers and also optionally derivatised cellulose ethers and silicones. Furthermore, the polymers as described in EP 1093796 (pages 3-8, paragraphs 17-68) may be used.

*Biogenic Active Ingredients*

Biogenic active ingredients are to be understood as meaning, for example, tocopherol, tocopherol acetate, tocopherol palmitate, ascorbic acid, deoxyribonucleic acid, retinol, bisabolol, allantoin, phytantriol, panthenol, AHA acids, amino acids, ceramides, pseudoceramides, essential oils, plant extracts and vitamin complexes.

*Antioxidants*

In addition to the primary light-protective substances it is also possible to use secondary light-protective substances of the antioxidant kind that interrupt the photochemical reaction chain triggered when UV radiation penetrates the skin or hair. Typical examples of such antioxidants are amino acids (e.g. glycine, histidine, tyrosine, tryptophan) and derivatives thereof, imidazoles (e.g. urocanic acid) and derivatives thereof, peptides, such as D,L-carnosine, D-carnosine, L-carnosine and derivatives thereof (e.g. anserine), carotenoids, carotenes, lycopene and derivatives thereof, chlorogenic acid and derivatives thereof, lipoic acid and derivatives thereof (e.g. dihydrolipoic acid), aurothioglycose, propylthiouracil and other thiols (e.g. thioredoxin, glutathione, cysteine, cystine, cystamine and the glycosyl, N-acetyl, methyl, ethyl, propyl, amyl, butyl, lauryl, palmitoyl, oleyl, linoleyl, cholesteryl and glyceryl esters thereof) and also salts thereof, dilauryl thiodipropionate, distearyl thiodipropionate, thiodipropionic acid and derivatives thereof (esters, ethers, peptides, lipids, nucleotides, nucleosides and salts) and also sulfoximine compounds (e.g. buthionine sulfoximines, homocysteine sulfoximine, buthionine sulfones, penta-, hexa-, hepta-thionine sulfoximine), also (metal) chelating agents (e.g. hydroxy fatty acids, palmitic acid phytic acid, lactoferrin), hydroxy acids (e.g. citric acid, lactic acid, malic acid), humic acid, bile acid, bile extracts, bilirubin, biliverdin, EDTA, EDDS, EGTA and derivatives thereof, unsaturated fatty acids and derivatives thereof (e.g. linolenic acid, linoleic acid, oleic acid), folic acid and derivatives thereof, ubiquinone and ubiquinol and derivatives thereof, vitamin C and derivatives (e.g. ascorbyl palmitate, magnesium ascorbyl phosphate, ascorbyl acetate), tocopherols and derivatives (e.g. vitamin E acetate), vitamin A and derivatives (e.g. vitamin A palmitate) and also coniferyl benzoate of benzoin resin, rutinic acid and derivatives thereof, glycosylrutin, ferulic acid, furfurylidene glucitol, carnosine, butyl hydroxytoluene, butyl hydroxyanisole, nordihydroguaiaretic acid, trihydroxybutyrophenone, uric acid and derivatives thereof, mannose and derivatives thereof, superoxide dismutase, N-[3-(3,5-di-tert-butyl-4-hydroxyphenyl)propionyl]sulfanilic acid (and salts thereof, for example the disodium salts), selenium and derivatives thereof (e.g. selenium methionine), stilbene and derivatives thereof (e.g. stilbene oxide, trans-stilbene oxide) and the derivatives suitable according to the invention (salts, esters, ethers, sugars, nucleotides, nucleosides, peptides and lipids) of those mentioned active ingredients. HALS (="Hindered Amine Light Stabilizers") compounds may also be mentioned. The amount of antioxidants present is usually from 0.001 to 30% by weight, preferably from 0.01 to 3% by weight, based on the weight of the sunscreen composition.

Particularly preferred antioxidants are those of the Tinogard® line available from BASF. For example, Tinogard® TT (pentaaerythrityl tetra-di-t-butyl hydroxyhydrocinnamate) and Tinogard® TL (Benzotriazolyl Dodecyl p-Cresol)

*Hydrotropic Agents*

To improve the flow behaviour it is also possible to employ hydrotropic agents, for example ethoxylated or non-ethoxylated mono-alcohols, diols or polyols with a low number of carbon atoms or their ethers (e.g. ethanol, isopropanol, 1,2-dipropanediol, propyleneglycol, glyerin, ethylene glycol, ethylene glycol monoethylether, ethylene glycol monobutylether, propylene glycol monomethylether, propylene glycol monoethylether, propylene glycol monobutylether, diethylene glycol monomethylether, diethylene glycol monoethylether, diethylene glycol monobutylether and similar products). The polyols that come into consideration for that purpose have preferably from 2 to 15 carbon atoms and at least two hydroxy groups. The polyols may also contain further functional groups, especially amino groups, and/or may be modified with nitrogen. Typical examples are as follows: glycerol, alkylene glycols, for example ethylene glycol, diethylene glycol, propylene glycol, butylene glycol, hexylene glycol and also polyethylene glycols having an average molecular weight of from 100 to 1000 Dalton, technical oligoglycerol mixtures having an intrinsic degree of condensation of from 1.5 to 10, for example technical diglycerol mixtures having a diglycerol content of from 40 to 50% by weight, methylol compounds, such as, especially, trimethylolethane, trimethylolpropane, trimethylolbutane, pentaerythritol and dipentaerythritol, lower alkyl-glucosides, especially those having from 1 to 8 carbon atoms in the alkyl radical, for example methyl and butyl glucoside, sugar alcohols having from 5 to 12 carbon atoms, for example sorbitol or mannitol, sugars having from 5 to 12 carbon atoms, for example glucose or saccharose, amino sugars, for example glucamine, dialcohol amines, such as diethanolamine or 2-amino-1,3-propanediol.

*Preservatives and Bacteria-Inhibiting Agents*

Suitable preservatives include, for example, Methyl-, Ethyl-, Propyl-, Butyl-parabens, Benzalkonium chloride, 2-Bromo-2-nitro-propane-1,3-diol, Dehydroacetic acid, Diazolidinyl Urea, 2-Dichloro-benzyl alcohol, DMDM hydantoin, Formaldehyde solution, Methyldibromoglutanitrile, Phenoxyethanol, Sodium Hydroxymethylglycinate, Imidazolidinyl Urea, Triclosan and further substance classes listed in the following reference: K. F. DePolo—A short textbook of cosmetology, Chapter 7, Table 7-2, 7-3, 7-4 and 7-5, p 210-219.

*Bacteria-Inhibiting Agents*

Typical examples of bacteria-inhibiting agents are preservatives that have a specific action against gram-positive bacteria, such as 2,4,4'-trichloro-2'-hydroxydiphenyl ether, chlorhexidine (1,6-di(4-chlorophenyl-biguanido)hexane) or TCC (3,4,4'-trichlorocarbanilide). A large number of aromatic substances and ethereal oils also have antimicrobial properties. Typical examples are the active ingredients eugenol, menthol and thymol in clove oil, mint oil and thyme oil. A natural deodorising agent of interest is the terpene alcohol farnesol (3,7,11-trimethyl-2,6,10-dodecatrien-1-ol), which is present in lime blossom oil. Glycerol monolaurate has also proved to be a bacteriostatic agent. The amount of the additional bacteria-inhibiting agents present is usually from 0.1 to 2% by weight, based on the solids content of the preparations.

*Perfume Oils*

There may be mentioned as perfume oils mixtures of natural and/or synthetic aromatic substances. Natural aromatic substances are, for example, extracts from blossom (lilies, lavender, roses, jasmine, neroli, ylang-ylang), from stems and leaves (geranium, patchouli, petitgrain), from fruit (aniseed, coriander, carraway, juniper), from fruit peel (bergamot, lemons, oranges), from roots (mace, angelica, celery, cardamom, costus, iris, calmus), from wood (pinewood, sandalwood, guaiacum wood, cedarwood, rosewood), from herbs and grasses (tarragon, lemon grass, sage, thyme), from needles and twigs (spruce, pine, Scots pine, mountain pine), from resins and balsams (galbanum, elemi, benzoin, myrrh, olibanum, opoponax). Animal raw materials also come into consideration, for example civet and castoreum. Typical synthetic aromatic substances are, for example, products of the ester, ether, aldehyde, ketone, alcohol or hydrocarbon type. Aromatic substance compounds of the ester type are, for example, benzyl acetate, phenoxyethyl isobutyrate, p-tert-butylcyclohexyl acetate, linalyl acetate, dimethylbenzylcarbinyl acetate, phenylethyl acetate, linalyl benzoate, benzyl formate, ethylmethylphenyl glycinate, allylcyclohexyl propionate, styrallyl propionate and benzyl salicylate. The ethers include, for example, benzyl ethyl ether, the aldehydes include, for example, the linear alkanals having from 8 to 18 hydrocarbon atoms, citral, citronellal, citronellyl oxyacetaldehyde, cyclamen aldehyde, hydroxycitronellal, lilial and bourgeonal, the ketones include, for example, the ionones, isomethylionone and methyl cedryl ketone, the alcohols include, for example, anethol, citronellol, eugenol, isoeugenol, geraniol, linalool, phenyl ethyl alcohol and terpinol, and the hydrocarbons include mainly the terpenes and balsams. It is preferable, however, to use mixtures of various aromatic substances that together produce an attractive scent. Ethereal oils of relatively low volatility, which are chiefly used as aroma components, are also suitable as perfume oils, e.g. sage oil, camomile oil, clove oil, melissa oil, oil of cinnamon leaves, lime blossom oil, juniper berry oil, vetiver oil, olibanum oil, galbanum oil, labolanum oil and lavandin oil. Preference is given to the use of bergamot oil, dihydromyrcenol, lilial, lyral, citronellol, phenyl ethyl alcohol, hexyl cinnamaldehyde, geraniol, benzyl acetone, cyclamen aldehyde, linalool, boisambrene forte, ambroxan, indole, hedione, sandelice, lemon oil, tangerine oil, orange oil, allyl amyl glycolate, cyclovertal, lavandin oil, muscatel sage oil, damascone, bourbon geranium oil, cyclohexyl salicylate, vertofix coeur, iso-E-Super, Fixolide NP, evernyl, iraldein gamma, phenylacetic acid, geranyl acetate, benzyl acetate, rose oxide, romillat, irotyl and floramat alone or in admixture with one another.

*Colorants*

There may be used as colourants the substances that are suitable and permitted for cosmetic purposes, as compiled, for example, in the publication "Kosmetische Färbemittel" of the Farbstoffkommission der Deutschen Forschungsgemeinschaft, Verlag Chemie, Weinheim, 1984, pages 81 to 106. The colourants are usually used in concentrations of from 0.001 to 0.1% by weight, based on the total mixture.

*Additional UV Screening Agents*

Sun screening agents which may be combined with the zinc oxide particles in the sunscreen compositions described above would include a range of organic UV screening agents selected from the group consisting of 1(+/-)-1,7,7-trimethyl-3-[(4-methylphenyl)methylene]bicyclo-[2.2.1]heptan-2-one, p-methyl benzylidene camphor,1,7,7-trimethyl-3-(phenylmethylene)bicyclo[2.2.1]heptan-2-one, benzylidene camphor, (2-Hydroxy-4-methoxyphenyl)(4-methylphenyl)methanone, 2,4-dihydroxybenzophenone, 2,2',4,4'-tetrahydroxybenzophenone, 2-Hydroxy-4-methoxy benzophenone, 2-Hydroxy-4-methoxy benzophenone-5-sulfonic acid, 2,2'-dihydroxy-4,4'-dimethoxybenzophenone, 2,2'-Dihydroxy-4-methoxybenzophenone, Alpha-(2-oxoborn-3-ylidene)toluene-4-sulphonic acid and its salts, 1-[4-(1,1-dimethylethyl)phenyl]-3-(4-methoxyphenyl)propane-1,3-dione, Methyl N,N,N-trimethyl-4-[(4,7,7-trimethyl-3-oxobicyclo[2,2,1]-hept-2-ylidene)methyl]anilinium sulphate, 3,3,5-Trimethyl cyclohexyl-2-hydroxy benzoate, Isopentyl p-methoxycinnamate, Menthyl-o-aminobenzoate,2-Ethylhexyl 2-cyano,3,3-diphenylacrylate, 2-ethylhexyl 4-(dimethylamino)benzoate, 2-ethylhexyl 4-methoxycinnamate, 2-ethylhexyl salicylate, Benzoic acid,4,4',4"-(1,3,5-triazine-2,4,6-triyltriimino)tris-,tris(2-ethylhexyl)ester, 4-aminobenzoic acid, Benzoic acid, 4-amino-, ethyl ester, polymer with oxirane, 2-phenyl-1H-benzimidazole-5-sulphonic acid,2-Propenamide, N-[[4-[(4,7,7-trimethyl-3-oxobicyclo[2.2.1]hept-2-ylidene)methyl]phenyl]methyl]-, homopolymer. Triethanolamine salicylate, 3,3'-(1,4-phenylenedimethylene)bis[7,7-dimethyl-2-oxobicyclo[2.2.1]heptane-1 methanesulfonic acid], Titanium dioxide, 2,2'-Methylene-bis-[6-(2H-benzotriazol-2-yl)-4-(1,1,3,3-tetramethylbutyl)-phenol], Bis-Ethylhexyloxyphenol Methoxyphenyl Triazine,1H-Benzimidazole-4,6-disulfonic acid, 2,2'-(1,4-phenylene)bis-, disodium salt, Benzoic acid, 4,4'-[[6-[4-[[(1,1-dimethylethyl)amino]carbonyl]-phenyl]amino]1,3,5-triazine-2,4-diyl]diimino]bis-, Phenol, 2-(2H-benzotriazol-2-yl)-4-methyl-6-[2-methyl-3-[1,3,3,3-tetramethyl-1-[(trimethylsily)oxy]disiloxanyl]propyl]-,Dimethicodiethylbenzalmalonate, Benzenesulfonic acid, 3-(2H-benzotriazol-2-yl)-4-hydroxy-5-(1-methylpropyl)-, monosodium salt, Benzoic acid, 2-[4-(diethylamino)-2-hydroxybenzoyl]-, hexyl ester, 1-Dodecanaminium, N-[3-[[4-(dimethylamino)benzoyl]amino]-propyl]N,N-dimethyl-, salt with 4-methylbenzenesulfonic acid (1:1), 1-Propanaminium, N,N,N-trimethyl-3-[(1-oxo-3-phenyl-2-propenyl)amino]-, chloride, 1H-Benzimidazole-4,6-disulfonic acid, 2,2'-(1,4-phenylene)bis-, 1,3,5-Triazine, 2,4,6-tris(4-methoxyphenyl)-,1,3,5-Triazine, 2,4,6-tris[4-[(2-ethylhexyl)oxy]phenyl]-, 1-Propanaminium, 3-[[3-[3-(2H-benzotriazol-2-yl)-5-(1,1-dimethylethyl)-4-hydroxyphenyl]-1-oxopropyl]amino]-N,N-diethyl-N-methyl-, methyl sulfate (salt), 2-Propenoic acid, 3-(1H-imidazol-4-yl)-, Benzoic acid, 2-hydroxy-, [4-(1-methylethyl)phenyl]methyl ester, 1,2,3-Propanetriol, 1-(4-aminobenzoate), Benzeneacetic acid, 3,4-dimethoxy-a-oxo-, 2-Propenoic acid, 2-cyano-3,3-diphenyl-, ethyl ester, Anthralinic acid, p-menth-3-yl ester, 2,2'-bis(1,4-phenylene)-1H-benzimidazole-4,6-disulphonic acid mono sodium salt or Disodium phenyl dibenzimidazole tetrasulfonate, 1,3,5-Triazine-2,4,6-triamine and N,N'-bis[4-[5-(1,1-dimethylpropyl)-2-benzoxazolyl]phenyl]-N"-(2-ethylhexyl), ), benzotirazolyl dodecyl p-cresol (BDC), diethylhexyl 2,6-napthalate (DEHN), diethylhexyl syringylidene malonate (DEHSM), polyester-8 and butyloctyl salicylate.

## Methods of Use

Also described herein is a method of using an overall environmental hazard score of each of a plurality of ultraviolet (UV) filters to formulate a composition. The composition has an ecotoxicological value of from 0 to 350, preferably from 1.5 to 300 (taking into account the UVA protection provided by a given UV filter composition). The method includes determining an environmental profile of each of the plurality of UV filters, determining the overall environmental hazard score of each UV filter, preparing the composition containing at least two of the UV filters, and determining an ecotoxicological value of the composition.

In certain embodiments, the environmental profile of each UV filter is based on environmental fate data and ecotoxicological data of the UV filter. The environmental profile for each UV filter includes a rating for six environmental chapters selected from biodegradation, bioaccumulation, acute aquatic toxicity, chronic aquatic toxicity, chronic terrestrial toxicity, sediment toxicity and combinations thereof. Any UV filter having at least one rating as a) endocrine active, b) persistent/bioaccumulative/toxic (PBT) or c) very persistent very bioaccumulative (vPvB), would be excluded from the composition. The environmental profile includes a rating ranging from 0.25 to 1 for at least one of biodegradation or bioaccumulation, wherein a rating of 0.25 represents the best environmental behavior and a rating of 1.0 represents the poorest environmental behavior. The environmental profile includes a rating of at least 0.25 for at least one of acute aquatic toxicity, chronic aquatic toxicity, chronic terrestrial toxicity or sediment toxicity, wherein a rating of 0.25 represents the best environmental behavior. Determining the environmental profile may be based on at least one data source selected from experimental data, ecotoxicological data, environmental fate data, study reports, scientific-based literature, information from government authorities and qualitative structure activity relationship models. Determining the environmental profile can include evaluating experimental data for a UV filter and if no experimental data is available, then calculating or estimating data to substitute for the experimental data. If calculating or estimating data to substitute for the experimental data is impossible, then determining a best-case scenario and a worst-case scenario for the UV filter. Determining the environmental profile can include evaluating experimental data for a UV filter and assessing reliability of the experimental data, wherein the experimental data are assigned a value of 1 to 4, and wherein only data with a reliability of 1 or 2 are used for the environmental profile.

The method may further include ranking the plurality of UV filters based on the overall environmental hazard score. Furthermore, determining the ecotoxicological value of the composition can include multiplying the overall environmental hazard score for each UV filter by its respective concentration (by weight) in the composition and adding together the resulting values for each UV filter.

## ILLUSTRATIVE EXAMPLES

*Example 1: Evaluating individual UV Filters*

Several UV filters were evaluated using the methods described herein. If no or less data was available, then there was large variability in the results and large uncertainty (i.e., the ecotoxicological profile was poorly defined). If sufficient data was available, then there was low variability and low uncertainty (i.e., the ecotoxicological profile was better defined). The kill criteria for the UV filters were also considered as were their environmental weaknesses. Certain UV filters were indicated has having acute and/or chronic aquatic toxicity, bioaccumulation, and/or endocrine activity or disruption and some were indicated has having one or more weaknesses in these areas.

Table 1 presents the ecotoxicological values of sunscreens containing various UV filters. The SPF value of the sunscreen compositions ranged from 6 to 60 while various combinations of UV filter compositions at different concentrations (wt%) were evaluated. The UVA recommendation was always taken into account. In particular, while the Eco value (also referred to as Ecotox value) is provided for each UV composition based on the following equation,

$$EcoSun\ Pass\ value = a\ \frac{SPF}{[UV\ filters]}$$

the Eco value 1 (also referred to as Ecotox value 1) is provided for each UV composition based on the following equation:

$$EcoSun\ Pass\ value\ 1 = a\ \frac{SPF\ +UVA - PF}{[UV\ filters]}$$

wherein "a" corresponds to the ecotoxicological evaluation of the used filter system; "SPF calc." corresponds to the numerical value for the SPF obtained from a sunscreen simulator or from in vivo or in vitro studies or using the claimed SPF, and "UVA-PFcalc." is the numerical value for the UVA protection factor from a sunscreen simulator or from in vivo or in vitro studies or using the claimed UVA-PF. Further, "[UV filters]" refers to the UV filter concentration in weight-%.

**Table 1 - Sunscreen Formulation Ecotox Ranking**

| SPF | UV Composition | SPF Calc. | UVA Calc. | UVA Ratio | Filter conc. (wt%) | EcoSun Pass value (SPF), i.e. Eco value | EcoSun Pass value (SPF + UVA), i.e. Eco value 1 |
|---|---|---|---|---|---|---|---|
| 6 | 2.4% OMC - 0.5% MBBT | 6.1 | 2 | 3.01 | 2.9 | **158.7** | **208** |
| 10 | 3.1% OMC - 1.5% MBBT | 9.9 | 3.3 | 3 | 4.6 | **146** | **204** |
| 15 | 3.7% OMC - 3% MBBT | 15 | 5.4 | 2.79 | 6.7 | **147.4** | **208** |
| 20 | 4.2% OMC - 4.5% MBBT | 20.2 | 7.6 | 2.65 | 8.7 | **149.4** | **213** |
| 25 | 5% OMC - 5.5% MBBT | 24.9 | 9 | 2.78 | 10.5 | **152.3** | **214** |
| 30 | 5.5% OMC - 7% MBBT | 30.3 | 11.2 | 2.68 | 12.5 | **154.6** | **219** |
| 50 | 9% OMC - 10% MBBT - 1% DHHB | 50.4 | 17.4 | 2.87 | 20 | **159.5** | **223** |
| 60 | 10% OMC - 10% MBBT - 2.8% DHHB - 0.9%EHT | 60.3 | 20.5 | 2.92 | 23.7 | **157.5** | **224** |
| 6 | 5%OCR - 0.1%BMDBM | 6.2 | 2.1 | 2.82 | 5.1 | **40.5** | **65** |
| 10 | 8%OCR - 0.5%BMDBM | 10.3 | 3.5 | 2.88 | 8.5 | **39.5** | **64** |
| 15 | 6%OCR - 2%BMDBM - 1% BZ-3 - 1% DBT | 15.4 | 6.6 | 2.26 | 10 | **41.3** | **83** |
| 20 | 6%OCR - 3%BMDBM - 2% BZ-3 - 2% DBT | 20.4 | 7.5 | 2.68 | 12.3 | **41.1** | **86** |
| 25 | 9%OCR - 3%BMDBM - 2% BZ-3 - 2% DBT | 25.9 | 8.7 | 2.87 | 16 | **41.6** | **81** |
| 30 | 10%OCR - 5%BMDBM - 2% BZ-3 - 2% DBT | 30 | 13.3 | 2.25 | 19 | **44.7** | **85** |
| 50 | 10%OCR - 5%BMDBM - 3% BEMT - 4% BZ-3 - 4% DBT | 50.6 | 16.6 | 3.01 | 26 | **45.5** | **101** |
| 60 | 10%OCR - 5%BMDBM - 3% BEMT - 5% BZ-3 - 4% DBT - 10%ZnO | 60.7 | 20.1 | 2.99 | 37 | **49.3** | **87** |

Compositions ultimately could be labeled with their ecotoxicological value, where the higher the value the friendlier the UV composition for the environment).

The methods of ranking compositions containing one or more UV filters was implemented in a SunScreen Simulator as set forth in Table 2. It is noted that the SunScreen Simulator may also be referred to as at least a part of a computer system that may be used to carry out a method of determining the ecotoxicological value of a sunscreen composition by use of the computer system. The computer system configured to execute or run the SunScreen Simulator may be provided as a distributed computer system comprising a client computer device and/or a network-side computer device, wherein the computer devices may be configured to exchange data with each other. The computer system may comprise a data input and/or output means configured to provide a choice of at least two ultraviolet, UV, filters and at least one other ingredient. Further, the computer system may be configured to determine, by a data processing means, in response to choice feedback data generated upon the choice of the UV filters and/or the other ingredient via data input and/or output means, ecotoxicological value data comprising the ecotoxicological value of the composition. Further, the computer system may be configured to provide, by the data input and/or output means, the ecotoxicological value to a user of the computer system.

# Table 2 – Implementation of Ranking in a SunScreen Simulator

| | | | 2 | mg/cm² |

| INCI Designation (and Abbreviation) | Trade Name | Concentration of UV-Absorbers in % |
|---|---|---|
| Phenylbenzimidazol sulfonic acid (PBSA) | | |
| 4-Methylbenzylidene camphor (MBC) | | |
| Ethylhexyl triazone (EHT) | Uvinul T 150 | 3 % |
| Octocrylene (OCR) | | |
| Butyl methoxydibenzoylmethane (BMDBM) | | |
| Ethylhexyl salicylate (EHS) | | 5 % |
| Ethylhexyl dimethyl PABA (Padimate O) | | |
| Ethylhexyl methoxycinnamate (EHMC) | Uvinul MC80 | |
| Isoamyl p-methoxycinnamate (IMC) | | |
| Terephthalidene dicamphor sulfonic acid (TDSA) | | |
| Drometrizole trisiloxane (DTS) | | |
| Methylene bis-benzotriazolyl tetramethylbutylphenol (MBBT) | Tinosorb M (active)* | |
| Bis-ethylhexyloxyphenol methoxyphenyl triazine (BEMT) | Tinosorb S | 3 % |
| Benzophenone-3 (B3) | Uvinul M40 | |
| Benzophenone-4 (B4) | Uvinul MS40 | |

**Integrated Transmission**

**Integrated Extinction**

**Results**

| SPF (calculated): | 50,9 | SPF Int. Method: | 50 |
|---|---|---|---|
| UVA-PF (simulated PPD method): | 21,6 | JCIA: | PA++++ |
| UVA/UVB-Ratio: | 0,80 | Star Rating: | *** |

Titanium dioxide (TiO₂), oil

Menthyl Anthranilate (MA)

Zinc oxide (ZnO), oil    Z cote HP1

Diethylhexyl butamido triazone (DBT)

Benzylidene malonate polysiloxane (BMP)

Disodium Phenyl Dibenzimidazole Tetrasulfonate (DPDT)

Homomenthyl Salicylate (HMS)

Diethylamino Hydroxybenzoyl Hexyl Benzoate (DHHB)    Uvinul A Plus    7 %

Tris Biphenyl Triazine (TBT)    Tinosorb A2B    3 %

Bis-ethylhexyloxyphenol methoxyphenyl triazine (BEMT), aqua    Tinosorb S aqua

Titanium dioxide (TiO₂), water

Zinc oxide (ZnO), water    ZnO

| | |
|---|---|
| Critical Wavelength: | 374 |
| Australian Standard: | Yes |
| Overall UV-Filter concentration: | 21 % |

| UVA-Balance (DIN 67502) | | | |
|---|---|---|---|
| 0,00E+00 | 50.0 | Input labelled SPF (if available): | |
| UVA-PF (DIN): | 21.7 | **50** | |
| UVA-Balance: | 42 | | |
| UVA-PF (COLIPA): | 21,4 | COLIPA-Ratio: | 2.34 |
| | | COLIPA-Ratio: | 0.43 |

| EcoTox Results | |
|---|---|
| EcoSun Pass value (SPF) | 141.5 |
| EcoSun Pass value 1 (SPF + UVA) | 200.9 |

**Other UV composition examples:**

| Claimed SPF | | 20 | 20 | 30 | 30 | 50 | 50+ | 50+ |
|---|---|---|---|---|---|---|---|---|
| UVB filters | Uvinul® T150 PBSA TiO2 EHS | 2% 1.5% - - | 3% 1% - - | 3% 2% - - | 1.5% 3.0% - - | 3.5% 3.5% 1.5% - | 3.5% 4% 2.5% - | 3% 3.5% - - |
| UVA filter | Uvinul® A Plus | 2.5% | 2.5% | 4,5% | 5.0% | 8% | 9% | 7% |
| Broadspectrum | Tinosorb® S | 1.5% | 1.5% | 2% | 2.0% | 3% | 3.5% | 4% |
| Total Filter Concentration | | 7% | 8% | 11.5% | 11.5 % | 19% | 22.5% | 17% |
| UVA-PF in vitro UVA-PF / SPF (Colipa) | | 7.2 >1/3 | 7.0 >1/3 | 10.6 >1/3 | 11.7 >1/3 | 17.7 >1/3 | 21.2 >1/3 | 23.1 >1/3 |
| SPF in vivo | | 21 (calc.) | 24 | 34 47 (UV10 107-1-8) | 34 | 55 | 73 (UV09 102-4-14) | 67 |
| Ecotox value | | 134.3 | 134.2 | 132.3 | 127.5 | 139.6 | 143.9 | 134.9 |
| Ecotox value 1 | | 212 | 205 | 212 | 219 | 218 | 219 | 226 |
| With Kill criteria | | 0 | 0 | 0 | 0 | 0 | 0 | 0 |

| Claimed SPF | | 20 UV09071-4-1 | 30 UV09071-1-2 UV09071-1-12 UV12103-1-5 | 50 UV09071-2-13 UV12103-2-3 UV11082-1-1 | 50+ UV09071-3-7 UV12103-3-2 |
|---|---|---|---|---|---|
| UVB | Uvinul®T 150 Tinosorb®A2 B | 2.6 4.0 | 3.0 6.0 | 3.2 8.0 | 3.4 9.0 |
| UV A | Uvinul®A Plus | 3.0 | 4.0 | 5.2 | 6.2 |
| B-S | Tinosorb®S | 0.8 | 1.0 | 2.5 | 2.5 |
| UVA-PF in vitro a.i. SPF / UVA-PF | | 7.5 >1/3 | 11.4 >1/3 | 18.0 >1/3 | 21.0 >1/3 |
| SPF in vivo | | 25.7 | 32.2 - WR* 62% UV09071-1-2 34.6 - WR* 57% UV09071-1-12 43.0 UV12103-1-4 | 60.4 UV09071-2-13 44.5 UV11082-1-1 45.0 UV12103-2-3 45.3 UV12103-2-4 | 58.4 UV09071-3-7 |
| Ecotox value | | 156.5 | 164 | 169.9 | 169.9 |
| Ecotox value 1 | | 222 | 234 | 253 | 257 |

| Claimed SPF | 30 UV-DE-15-124-3-1 UV-DE-17-072-2-1 | 50 UV-DE-15-124-4-1 UV-DE-17-042-4-3 UV-DE-17-099-2-6 | 50 UV-DE-17-042-2-2 | 50+ |
|---|---|---|---|---|
| Uvinul® T 150 EHS | 1.5% 5.0% | 2.5% 5.0% | 2.5% 5.0% | 3.0% 5.0% |

(continued)

| Claimed SPF | 30<br>UV-DE-15-124-3-1<br>UV-DE-17-072-2-1 | 50<br>UV-DE-15-124-4-1<br>UV-DE-17-042-4-3<br>UV-DE-17-099-2-6 | 50<br>UV-DE-17-042-2-2 | 50+ |
|---|---|---|---|---|
| Tinosorb® A2B | 4.4% | 6.0% | 6.0% | 6.0% |
| Uvinul® A Plus | 3.0% | 4.0% | 6.0% | 7.% |
| Tinosorb® M<br>Tinosorb® S<br>Tinosorb® S Lite<br>Aqua | -<br>1.0%<br>-<br>- | 4.0%<br>1.0%<br>-<br>- | -<br>2.0%<br>-<br>- | -<br>1.0%<br>1.0%<br>10.0% |
| UVA-PF in silico<br>UVA-PF / SPF | 10.2 (in vitro)<br>> 1/3 | 17.7 (in vitro)<br>>1/3 | 20.7<br>>1/3 | 25.4<br>> 1/3 |
| SPF in silico | 30 (in vivo)<br>34 (in vivo) | 53 (in vivo) | 50<br>49 (in vivo) | 60 |
| Ecotox value | 135 | 147.8 | 141.3 | 141.5 |
| Ecotox value 1 | 206 | 230 | 224 | 236 |

| SPF calc | 30 | 50 |
|---|---|---|
| Uvinul® T 150<br>Tinosorb® A2B | 2.5% | 3%<br>8% |
| Uvinul® A Plus | 5% | 5% |
| Tinosorb® M<br>Tinosorb® S | 6%<br>3% | 8% |
| Ecotox value | 122.6 | 166.6 |
| Ecotox value 1 | 225 | 248 |

| Claimed SPF | 15 | 20 | 30 | 50 | 50+ |
|---|---|---|---|---|---|
| Uvinul® T 150<br>Tinosorb® A2B | 1.5%<br>2.0% | 1.5%<br>4.0% | 1.5%<br>6.0% | 2.0%<br>8.0% | 2.5%<br>8.0% |
| Tinosorb® S<br>Tinosorb® M | 1.0%<br>4.0% | 1.5%<br>6.0% | 1.7%<br>6.0% | 2.5%<br>12.0% | 2.0%<br>15.0% |
| UVA-PF<br>UVA-PF / SPF | 5.9<br>>1/3 | 8.0<br>> 1/3 | 10.3<br>>1/3 | 17.3<br>>1/3 | 19.9<br>> 1/3 |
| SPF in vivo | | | 35 | 57 | 64 |
| SPF calc.[1] | 18.1 | 27.8 | 33.7 | 54.4 | 59.8 |
| EcoSun Pass value (SPF) (with kill criteria) | 181 | 189 | 196 | 203 | 203 |
| Ecotox value 1[2] (with Kill criteria) | 240 | 244 | 256 | 268 | 271 |

| Claimed SPF | 30 | 30 | 50 | 50 |
|---|---|---|---|---|
| Titanium dioxide | - | 3.0% | - | 4.5% |
| Tinosorb® A2B | 5.0% | 8.0% | 16.0% | 10.0% |
| Tinosorb® M | - | - | 14.% | 14.0% |
| Z Cote HP1 | 13.1% | - | 5.1% | - |
| UVA-PF | 13 | 6.2 | 19.1 | 17.7 |
| UVA-PF / SPF | >1/3 | <1/3 | >1/3 | > 1/3 |
| SPF calc | 23.6 | 23 | 49 | 50.2 |
| SPF vivo | | 34 | | |
| EcoSun Pass value (SPF) (with kill criteria) | 0 | 173 | 0 | 167 |
| Ecotox value 1 (with kill criteria) | 0 | 220 | 0 | 226 |

| | | Minimal UVA protection 1/3 | Long UVA example 1 | Long UVA Example 2 |
|---|---|---|---|---|
| UV-filters | Uvinul T 150 | 4% | 0.5% | 2% |
| | EHS | 4% | 3.5% | - |
| | Tinosorb S | 2.5% | 1% | 2% |
| | Uvinul A Plus | 4% | 4% | 8% |
| | Tinosorb M | - | 8% | 6% |
| | Tinosorb A2B | - | 3% | - |
| | Total | 14.5% | 14.5% | 15% |
| Performance | SPF in silico | 29.8 | 29.7 | 29.7 |
| | UVA-PF in silico | 11 | 27.6 | 36.3 |
| EcoSun Pass value | EcoSun Pass value (SPF) | 126 | 123 | 107 |
| | EcoSun Pass value 1 (SPF + UVA) | 172 | 233 | 233 |
| EHS, ethylhexyl salycilate | | | | |

| | | Minimal UVA protection 1/3 | Increased UVA protection 1/2 | Long UVA |
|---|---|---|---|---|
| UV-filters | Uvinul T 150 | 4% | 4% | 2.5% |
| | Titanium dioxide (nano) | 1.5% | - | 1.5% |
| | Uvinul A Plus | 4% | 6.5% | 10% |
| | Total | 9.5% | 10.5% | 14% |
| Performance | SPF in silico | 22 | 21.9 | 21.9 |
| | UVA-PF in silico | 7.3 | 11.5 | 22.8 |
| EcoSun Pass value | EcoSun Pass value (SPF) | 115 | 100 | 75 |
| | EcoSun Pass value 1 (SPF + UVA) | 171 | 172 | 175 |

(continued)

| | Minimal UVA protection 1/3 | Long UVA | Minimal UVA protection 1/3 | Minimal UVA protection 1/3 | Minimal UVA protection 1/3 |
|---|---|---|---|---|---|
| Uvinul T 150 | 3% | 2.5% | - | - | 2.5% |
| EHS | 5% | - | 4.5% | 5% | 5% |
| Tinosorb S | 1.5% | 2% | 4% | 2% | |
| Uvinul A Plus | 6% | 10% | - | - | 8% |
| Tinosorb A2B | 6% | 4% | - | 6% | |
| Tinosorb M | - | 6% | - | - | 6% |
| HMS | - | - | 9.5% | - | |
| PBSA | - | - | 1% | - | |
| BMDBM | - | - | 4.7% | 4% | |
| Uvinul N539T | - | - | - | 10% | |
| Uvinul MC80 | - | - | - | - | 10% |
| Total | 18.5% | 19.5% | 23.7% | 24% | 28.5% |
| SPF in silico | 50.1 | 50.1 | 40.5 | 50.6 | 53.5 |
| UVA-PF in silico | 19.5 | 44.6 | 12.9 | 20.2 | 16.9 |
| EcoSun Pass value (SPF) (with kill criteria) | 159 | 134 | 0 | 0 | 114 |
| EcoSun Pass value (SPF + UVA) (with kill criteria) | 221 | 255 | 0 | 0 | 166 |
| PBSA: phenylbenzimidazole sulfonic acid; BMDBM: Butyl Methoxydibenzoylmethane; HMS: Homosalate; | | | | | |

## Claims

1. A method of formulating a composition, comprising:

determining an environmental profile of each of a plurality of ultraviolet (UV) filters;
determining an overall environmental hazard score of each UV filter and wherein each of the UV filters is selected to have an overall environmental hazard score of 1.5 to 7.0;
preparing the composition comprising at least two of the UV filters and at least one other ingredient;
determining an ecotoxicological evaluation of the used filter system "a" of the composition, and
determining an ecotoxicological value (EcoSun Pass value) of the composition,
wherein the ecotoxicological value ranges from 0 to 350,
wherein the overall environmental hazard score is a sum of ratings for six environmental chapters, wherein the environmental chapters selected from a group consisting of biodegradation, bioaccumulation, acute aquatic toxicity, chronic aquatic toxicity, chronic terrestrial toxicity, and sediment toxicity, and wherein the environmental profile of each UV filter is based on environmental fate data and ecotoxicological data of the UV filter, wherein the environmental profile for each UV filter comprises a rating for each environmental chapter selected from a group consisting of biodegradation, bioaccumulation, acute aquatic toxicity, chronic aquatic toxicity, chronic terrestrial toxicity, and sediment toxicity, and
wherein determining the ecotoxicological evaluation of the used filter system "a" of the composition comprises multiplying the overall environmental hazard score for each UV filter by its respective concentration (by weight) in the composition and adding together the resulting values for each UV filter;
wherein the ecotoxicological value (EcoSun Pass value) is represented as

$$EcoSun\ Pass\ value = a\frac{SPF\ +\ UVA - PF}{[UV\ filters]}$$

wherein "a" corresponds to the ecotoxicological evaluation of the used filter system; "SPF" corresponds to the numerical value for the SPF obtained from a sunscreen simulator or obtained from in vivo or in vitro studies or using the claimed SPF, "UVA-PF" is the numerical value for the UVA protection factor from a sunscreen simulator or from in vivo or in vitro studies or using the claimed UVA-PF, and "[UV filters]" refers to the UV filter concentration in weight-%, and

wherein the ratings for the environmental chapters are assessed as follows:

### I. Biodegradation (According to OECD criteria)

| | |
|---|---|
| Readily biodegradable/not persistent | Factor 0.25 |
| Biodegradable/ not persistent | Factor 0.5 |
| Partly biodegradable/potential persistent | Factor 0.75 |
| Poorly biodegradable/persistent | Factor 1 |

### II. Bioaccumulation

| | | |
|---|---|---|
| Bioaccumulation factor (BCF) | < 500 | Factor 0.25 |
| Bioaccumulation factor (BCF) | >= 500 - < 2000 | Factor 0.5 |
| Bioaccumulation factor (BCF) | >= 200 - < 5000 | Factor 0.75 |
| Bioaccumulation factor (BCF) | > 5000 | Factor 1.0 |

### III. Acute aquatic toxicity

| | |
|---|---|
| EC50 > 100 mg/L or > W | Factor 0.25 |
| EC50 $\leq$ 100 $\geq$ 10 mg/L | Factor 0.5 |
| EC50 < 10 $\geq$ 1 mg/L | Factor 0.75 |
| EC50 < 1 mg/L | Factor 1 |
| EC50 10 times lower as previously | + 0.25 |

### IV. Chronic aquatic toxicity

| | |
|---|---|
| NOEC/EC10 $\geq$ 10 mg/L | Factor 0.25 |
| NOEC/EC10 < 10 $\geq$ 1 mg/L | Factor 0.5 |
| NOEC/EC10 < 1 $\geq$ 0.1 mg/L | Factor 0.75 |
| NOEC/EC10 < 0.1 $\geq$ 0.01 mg/L | Factor 1 |
| NOEC/EC10 10 times lower | + 0.25 |

### V. Chronic terrestrial toxicity

| | |
|---|---|
| NOEC/EC10 $\geq$ 1000 mg/kg | Factor 0.25 |
| NOEC/EC10 < 1000 $\geq$ 100 mg/kg | Factor 0.5 |
| NOEC/EC10 < 100 $\geq$ 10 mg/kg | Factor 0.75 |
| NOEC/EC10 < 10 $\geq$ 1 mg/kg | Factor 1 |
| NOEC/EC10 10 times lower | + 0.25 |

### VI. Sediment toxicity

NOEC/EC10 $\geq$ 1000 mg/kg    Factor 0.25
NOEC/EC10 < 1000 $\geq$ 100 mg/kg    Factor 0.5
NOEC/EC10 < 100 $\geq$ 10 mg/kg    Factor 0.75
NOEC/EC10 < 10 $\geq$ 1 mg/kg    Factor 1
NOEC/EC10 10 times lower    + 0.25.

**2.** The method of claim 1, wherein the at least one other ingredient forms a base of a sunscreen composition, preferably wherein the at least one other ingredient is selected from a group consisting of a surfactant, super-fatting agent, oil, emulsifier, wax, consistency regulator, thickener, polymers, silicon, siloxane, stabilizer, biogenic active ingredient, natural or synthetic triglyceride, fatty acid, fatty alcohol, ester of fatty acid, swelling agent, further UV light-protective agent, antioxidant, hydrotropic agent, preservative, solubiliser, perfume oil, colorant, bacteria-inhibiting agent, adjuvant and mixtures thereof.

**3.** The method of claim 1 or 2,

wherein the environmental profile comprises a rating ranging from 0.25 to 1 for at least one of biodegradation or bioaccumulation, wherein a rating of 0.25 represents the best environmental behavior and a rating of 1.0 represents the poorest environmental behavior and/or
wherein the environmental profile comprises a rating of at least 0.25 for at least one of acute aquatic toxicity, chronic aquatic toxicity, chronic terrestrial toxicity or sediment toxicity, wherein a rating of 0.25 represents the best environmental behavior.

**4.** The method of any one of claims 1 to 3, wherein determining the environmental profile is based on at least one data source selected from a group consisting of experimental data, ecotoxicological data, environmental fate data, study reports, scientific-based literature, information from government authorities and qualitative structure activity relationship models, and/or

wherein determining the environmental profile comprises evaluating experimental data for a UV filter and if no experimental data is available, then calculating or estimating data to substitute for the experimental data, preferably wherein if calculating or estimating data to substitute for the experimental data is impossible, then determining a best-case scenario and a worst-case scenario for the UV filter, and/or
wherein determining the environmental profile comprises evaluating experimental data for a UV filter and assessing reliability of the experimental data, wherein the experimental data are assigned a value of 1 to 4, and wherein only data with a reliability of 1 or 2 are used for the environmental profile.

**5.** The method of any one of claims 1 to 4, further comprising ranking the plurality of UV filters based on the overall environmental hazard score.

**6.** A method of using an overall environmental hazard score of each of a plurality of ultraviolet (UV) filters to formulate a composition comprising an ecotoxicological value (EcoSun Pass value) of 0 to 350, the method comprising:

determining an environmental profile of each of the plurality of UV filters;
determining the overall environmental hazard score of each UV filter;
preparing the composition comprising at least two of the UV filters;
determining an ecotoxicological evaluation of the used filter system "a" of the composition, and
determining an ecotoxicological value of the composition, wherein the environmental profile of each UV filter is based on environmental fate data and ecotoxicological data of the UV filter,
wherein the overall environmental hazard score is a sum of ratings for six environmental chapters, wherein the environmental chapters are selected from a group consisting of biodegradation, bioaccumulation, acute aquatic toxicity, chronic aquatic toxicity, chronic terrestrial toxicity, and sediment toxicity,
wherein determining the ecotoxicological evaluation of the used filter system "a" of the composition comprises multiplying the overall environmental hazard score for each UV filter by its respective concentration (by weight) in the composition and adding together the resulting values for each UV filter,
wherein the environmental profile for each UV filter comprises a rating for each environmental chapter selected from a group consisting of biodegradation, bioaccumulation, acute aquatic toxicity, chronic aquatic toxicity, chronic terrestrial toxicity, and sediment toxicity,
wherein the EcoSun Pass value is represented as

$$EcoSun\ Pass\ value = a\frac{SPF\ +\ UVA - PF}{[UV\ filters]}$$

wherein "a" corresponds to the ecotoxicological evaluation of the used filter system; "SPF" corresponds to the numerical value for the SPF obtained from a sunscreen simulator or obtained from in vivo or in vitro studies or using the claimed SPF, "UVA-PF" is the numerical value for the UVA protection factor from a sunscreen simulator or from in vivo or in vitro studies or using the claimed UVA-PF, and "[UV filters]" refers to the UV filter concentration in weight-%,

wherein the environmental profile comprises a rating ranging from 0.25 to 1 for biodegradation or bioaccumulation, wherein a rating of 0.25 represents the best environmental behavior and a rating of 1.0 represents the poorest environmental behavior, and

wherein the ratings for the environmental chapters are assessed as follows:

I. Biodegradation (According to OECD criteria)

| | |
|---|---|
| Readily biodegradable/not persistent | Factor 0.25 |
| Biodegradable/ not persistent | Factor 0.5 |
| Partly biodegradable/potential persistent | Factor 0.75 |
| Poorly biodegradable/persistent | Factor 1 |

II. Bioaccumulation

| | | |
|---|---|---|
| Bioaccumulation factor (BCF) | < 500 | Factor 0.25 |
| Bioaccumulation factor (BCF) | >= 500 - < 2000 | Factor 0.5 |
| Bioaccumulation factor (BCF) | >= 200 - < 5000 | Factor 0.75 |
| Bioaccumulation factor (BCF) | > 5000 | Factor 1.0 |

III. Acute aquatic toxicity

| | |
|---|---|
| EC50 > 100 mg/L or > W | Factor 0.25 |
| EC50 $\leq$ 100 $\geq$ 10 mg/L | Factor 0.5 |
| EC50 < 10 $\geq$ 1 mg/L | Factor 0.75 |
| EC50 < 1 mg/L | Factor 1 |
| EC50 10 times lower as previously | + 0.25 |

IV. Chronic aquatic toxicity

| | |
|---|---|
| NOEC/EC10 $\geq$ 10 mg/L | Factor 0.25 |
| NOEC/EC10 < 10 $\geq$ 1 mg/L | Factor 0.5 |
| NOEC/EC10 < 1 $\geq$ 0.1 mg/L | Factor 0.75 |
| NOEC/EC10 < 0.1 $\geq$ 0.01 mg/L | Factor 1 |
| NOEC/EC10 10 times lower | + 0.25 |

V. Chronic terrestrial toxicity

| | |
|---|---|
| NOEC/EC10 $\geq$ 1000 mg/kg | Factor 0.25 |
| NOEC/EC10 < 1000 $\geq$ 100 mg/kg | Factor 0.5 |
| NOEC/EC10 < 100 $\geq$ 10 mg/kg | Factor 0.75 |

(continued)

| NOEC/EC10 < 10 ≥ 1 mg/kg | Factor 1 |
|---|---|
| NOEC/EC10 10 times lower | + 0.25 |

VI. Sediment toxicity

| NOEC/EC10 ≥ 1000 mg/kg | Factor 0.25 |
|---|---|
| NOEC/EC10 < 1000 ≥ 100 mg/kg | Factor 0.5 |
| NOEC/EC10 < 100 ≥ 10 mg/kg | Factor 0.75 |
| NOEC/EC10 < 10 ≥ 1 mg/kg | Factor 1 |
| NOEC/EC10 10 times lower | + 0.25. |

**7.** The method of claim 6, wherein the environmental profile comprises a rating of at least 0.25 for acute aquatic toxicity, chronic aquatic toxicity, chronic terrestrial toxicity or sediment toxicity, wherein a rating of 0.25 represents the best environmental behavior.

**8.** The method of claim 6 or 7, wherein determining the environmental profile is based on at least one data source selected from a group consisting of experimental data, ecotoxicological data, environmental fate data, study reports, scientific-based literature, information from government authorities and qualitative structure activity relationship models, and/or

wherein determining the environmental profile comprises evaluating experimental data for a UV filter and if no experimental data is available, then calculating or estimating data to substitute for the experimental data, preferably wherein if calculating or estimating data to substitute for the experimental data is impossible, then determing a best-case scenario and a worst-case scenario for the UV filter, and/or
wherein determining the environmental profile comprises evaluating experimental data for a UV filter and assessing reliability of the experimental data, wherein the experimental data are assigned a value of 1 to 4, and wherein only data with a reliability of 1 or 2 are used for the environmental profile.

**9.** The method of any one of claim 6 to 8, further comprising ranking the plurality of UV filters based on the overall environmental hazard score.

**Patentansprüche**

**1.** Verfahren zur Formulierung einer Zusammensetzung, umfassend:

Bestimmen eines Umweltprofils jedes einer Mehrzahl von Ultraviolettfiltern (UV-Filtern);
Bestimmen einer Umweltgefährdungsgesamtnote jedes UV-Filters und wobei jeder der Filter so ausgewählt wird, dass er eine Umweltgefährdungsgesamtnote von 1,5 bis 7,0 aufweist;
Herstellen der Zusammensetzung, die mindestens zwei der UV-Filter und mindestens einen anderen Bestandteil umfasst;
Bestimmen einer ökotoxikologischen Evaluierung des verwendeten Filtersystems "a" der Zusammensetzung und Bestimmen eines ökotoxikologischen Wertes (EcoSun-Pass-Wert) der Zusammensetzung,
wobei der ökotoxikologische Wert im Bereich von 0 bis 350 liegt,
wobei die Umweltgefährdungsgesamtnote eine Summe von Bewertungen für sechs Umweltkapitel ist, wobei die Umweltkapitel ausgewählt werden aus einer Gruppe bestehend aus biologischem Abbau, Bioakkumulation, akuter aquatischer Toxizität, chronischer aquatischer Toxizität, chronischer terrestrischer Toxizität und Sedimenttoxizität, und wobei das Umweltprofil jedes UV-Filters auf Daten zum Umweltverhalten und ökotoxikologischen Daten des UV-Filters basiert, wobei das Umweltprofil für jeden UV-Filter eine Bewertung für jedes Umweltkapitel umfasst, das ausgewählt wird aus einer Gruppe bestehend aus biologischem Abbau, Bioakkumulation, akuter aquatischer Toxizität und chronischer aquatischer Toxizität, chronischer terrestrischer Toxizität und Sedimenttoxizität, und
wobei das Bestimmen der ökotoxikologischen Evaluierung des verwendeten Filtersystems "a" der Zusammensetzung ein Multiplizieren der Umweltgefährdungsgesamtnote für jeden UV-Filter mit seiner jeweiligen Konzentration (bezogen auf Gewicht) in der Zusammensetzung und Zusammenzählen der resultierenden Werte für jeden UV-Filter umfasst;
wobei der ökotoxikologisch Wert (EcoSun-Pass-Wert) dargestellt wird als

$$EcoSun - Pass - Wert = a\frac{SPF + UVA - PF}{[UV - Filter]}$$

wobei "a" der ökotoxikologischen Evaluierung des verwendeten Filtersystems entspricht;
"SPF" dem numerischen Wert für den SPF entspricht, der erhalten wird aus einem Sonnenschutzsimulator oder aus In-vivo- oder In-vitro-Studien oder unter Verwendung des beanspruchten SPF, "UVA-PF" der numerische Wert für den UVA-Schutzfaktor aus einem Sonnenschutzsimulator oder aus In-vivo- oder In-vitro-Studien oder unter Verwendung des beanspruchten UVA-PF ist und "[UV-Filter]" sich auf die UV-Filterkonzentration in Gewichts% bezieht, und
wobei die Bewertungen für die Umweltkapitel beurteilt werden, wie folgt:

I. Biologischer Abbau (gemäß OECD-Kriterien)

| | |
|---|---|
| Leicht biologisch abbaubar/nicht persistent | Faktor 0,25 |
| Biologisch abbaubar/nicht persistent | Faktor 0,5 |
| Teilweise biologisch abbaubar/potenziell persistent | Faktor 0,75 |
| Schlecht biologisch abbaubar/persistent | Faktor 1 |

II. Bioakkumulation

| | | |
|---|---|---|
| Bioakkumulationsfaktor (BCF) | < 500 | Faktor 0,25 |
| Bioakkumulationsfaktor (BCF) | >= 500 - < 2000 | Faktor 0,5 |
| Bioakkumulationsfaktor (BCF) | >= 200 - < 5000 | Faktor 0,75 |
| Bioakkumulationsfaktor (BCF) | > 5000 | Faktor 1,0 |

III. Akute aquatische Toxizität

| | |
|---|---|
| EC50 > 100 mg/l oder > W | Faktor 0,25 |
| EC50 $\leq$ 100 $\geq$ 10 mg/l | Faktor 0,5 |
| EC50 < 10 $\geq$ 1 mg/l | Faktor 0,75 |
| EC50 < 1 mg/l | Faktor 1 |
| EC50 10-mal niedriger als zuvor | + 0,25 |

IV. Chronische aquatische Toxizität

| | |
|---|---|
| NOEC/EC10 $\geq$ 10 mg/l | Faktor 0,25 |
| NOEC/EC10 < 10 $\geq$ 1 mg/l | Faktor 0,5 |
| NOEC/EC10 < 1 $\geq$ 0,1 mg/l | Faktor 0,75 |
| NOEC/EC10 < 0,1 $\geq$ 0,01 mg/l | Faktor 1 |
| NOEC/EC10 10-mal niedriger | + 0,25 |

V. Chronische terrestrische Toxizität

| | |
|---|---|
| NOEC/EC10 $\geq$ 1000 mg/kg | Faktor 0,25 |
| NOEC/EC10 < 1000 $\geq$ 100 mg/kg | Faktor 0,5 |
| NOEC/EC10 < 100 $\geq$ 10 mg/kg | Faktor 0,75 |
| NOEC/EC10 < 10 $\geq$ 1 mg/kg | Faktor 1 |
| NOEC/EC10 10-mal niedriger | + 0,25 |

VI. Sedimenttoxizität

| NOEC/EC10 $\geq$ 1000 mg/kg | Faktor 0,25 |
| NOEC/EC10 < 1000 $\geq$ 100 mg/kg | Faktor 0,5 |
| NOEC/EC10 < 100 $\geq$ 10 mg/kg | Faktor 0,75 |
| NOEC/EC10 < 10 $\geq$ 1 mg/kg | Faktor 1 |
| NOEC/EC10 10-mal niedriger | 0,25. |

**2.** Verfahren nach Anspruch 1, wobei der mindestens eine andere Bestandteil eine Basis einer Sonnenschutzzusammensetzung bildet, wobei vorzugsweise der mindestens eine Bestandteil ausgewählt wird aus einer Gruppe bestehend aus einem Tensid, einem Überfettungsmittel, Öl, einem Emulgator, Wachs, einem Konsistenzregler, einem Verdickungsmittel, Polymeren, Silizium, Siloxan, einem Stabilisator, einem biogenen Wirkstoff, einem natürlichen oder synthetischen Triglycerid, Fettsäure, Fettalkohol, einem Fettsäureester, einem Quellmittel, weiteren UV-Lichtschutzmitteln, einem Antioxidans, einem hydrotropen Mittel, einem Konservierungsmittel, einem Lösungsvermittler, Parfümöl, einem Farbstoff, einem bakterienhemmenden Mittel, einem Adjuvans und Mischungen davon.

**3.** Verfahren nach Anspruch 1 oder 2,

wobei das Umweltprofil eine Bewertung im Bereich von 0,25 bis 1 für mindestens eines von biologischem Abbau oder Bioakkumulation umfasst, wobei eine Bewertung von 0,25 das beste Umweltverhalten darstellt und eine Bewertung von 1,0 das schlechteste Umweltverhalten darstellt, und/oder

wobei das Umweltprofil eine Bewertung von mindestens 0,25 für mindestens eines von akuter aquatischer Toxizität, chronischer aquatischer Toxizität, chronischer terrestrischer Toxizität oder Sedimenttoxizität umfasst, wobei eine Bewertung von 0,25 das beste Umweltverhalten darstellt.

**4.** Verfahren nach einem der Ansprüche 1 bis 3, wobei das Bestimmen des Umweltprofils auf mindestens einer Datenquelle basiert, die ausgewählt wird aus einer Gruppe bestehend aus experimentellen Daten, ökotoxikologischen Daten, Daten zum Umweltverhalten, Studienberichten, wissenschaftlich fundierter Literatur, Informationen von Regierungsbehörden und qualitativen Struktur-Aktivitäts-Beziehungsmodellen, und/oder

wobei das Bestimmen des Umgebungsprofils ein Evaluieren experimenteller Daten für einen UV-Filter und, wenn keine experimentellen Daten verfügbar sind, dann ein Berechnen oder Schätzen von Daten als Ersatz für die experimentellen Daten umfasst, wobei dann, wenn das Berechnen oder Schätzen von Daten als Ersatz für die experimentellen Daten unmöglich ist, vorzugsweise ein Best-Case-Szenario und ein Worst-Case-Szenario für den UV-Filter bestimmt wird, und/oder

wobei das Bestimmen des Umweltprofils ein Evaluieren experimenteller Daten für einen UV-Filter und Beurteilen der Zuverlässigkeit der experimentellen Daten umfasst, wobei den experimentellen Daten ein Wert von 1 bis 4 zugeordnet wird, und wobei für das Umweltprofil nur Daten mit einer Zuverlässigkeit von 1 oder 2 verwendet werden.

**5.** Verfahren nach einem der Ansprüche 1 bis 4, ferner umfassend ein Einstufen der Mehrzahl von UV-Filtern basierend auf der Umweltgefährdungsgesamtnote.

**6.** Verfahren zur Verwendung einer Umweltgefährdungsgesamtnote für jeden einer Mehrzahl von Ultraviolettfiltern (UV-Filtern) zum Formulieren einer Zusammensetzung mit einem ökotoxikologischen Wert (EcoSun-Pass-Wert) von 0 bis 350, wobei das Verfahren umfasst:

Bestimmen eines Umweltprofils jedes der Mehrzahl von UV-Filtern;
Bestimmen der Umweltgefährdungsgesamtnote jedes UV-Filters;
Herstellen der Zusammensetzung, die mindestens zwei der UV-Filter umfasst;
Bestimmen einer ökotoxikologischen Evaluierung des verwendeten Filtersystems "a" der Zusammensetzung und Bestimmen eines ökotoxikologischen Wertes der Zusammensetzung, wobei das Umweltprofil jedes UV-Filters auf Daten zum Umweltverhalten und ökotoxikologischen Daten des UV-Filters basiert,
wobei die Umweltgefährdungsgesamtnote eine Summe von Bewertungen für sechs Umweltkapitel ist, wobei die Umweltkapitel ausgewählt werden aus einer Gruppe bestehend aus biologischem Abbau, Bioakkumulation, akuter aquatischer Toxizität, chronischer aquatischer Toxizität, chronischer terrestrischer Toxizität und Sedimenttoxizität,

wobei das Bestimmen der ökotoxikologische Evaluierung des verwendeten Filtersystems "a" der Zusammensetzung ein Multiplizieren der Umweltgefährdungsgesamtnote für jeden UV-Filter mit seiner jeweiligen Konzentration (bezogen auf Gewicht) in der Zusammensetzung und Zusammenzählen der resultierenden Werte für jeden UV-Filter umfasst, wobei das Umweltprofil für jeden UV-Filter eine Bewertung für jedes Umweltkapitel umfasst, das ausgewählt wird aus einer Gruppe bestehend aus biologischem Abbau, Bioakkumulation, akuter aquatischer Toxizität und chronischer aquatischer Toxizität, chronischer terrestrischer Toxizität und Sedimenttoxizität,

wobei der EcoSun-Pass-Wert dargestellt wird als

$$EcoSun - Pass - Wert = a \frac{SPF + UVA - PF}{[UV - Filter]}$$

wobei "a" der ökotoxikologischen Evaluierung des verwendeten Filtersystems entspricht;

"SPF" dem numerischen Wert für den SPF entspricht, der erhalten wird aus einem Sonnenschutzsimulator oder aus In-vivo- oder In-vitro-Studien oder unter Verwendung des beanspruchten SPF, "UVA-PF" der numerische Wert für den UVA-Schutzfaktor aus einem Sonnenschutzsimulator oder

aus In-vivo- oder In-vitro-Studien oder unter Verwendung des beanspruchten UVA-PF ist und "[UV-Filter]" sich auf die UV-Filterkonzentration in Gewichts% bezieht, und

wobei das Umweltprofil eine Bewertung im Bereich von 0,25 bis 1 für biologischen Abbau oder Bioakkumulation umfasst, wobei eine Bewertung von 0,25 das beste Umweltverhalten darstellt und eine Bewertung von 1,0 das schlechteste Umweltverhalten darstellt, und

wobei die Bewertungen für die Umweltkapitel beurteilt werden, wie folgt:

### I. Biologischer Abbau (gemäß OECD-Kriterien)

| | |
|---|---|
| Leicht biologisch abbaubar/nicht persistent | Faktor 0,25 |
| Biologisch abbaubar/nicht persistent | Faktor 0,5 |
| Teilweise biologisch abbaubar/potenziell persistent | Faktor 0,75 |
| Schlecht biologisch abbaubar/persistent | Faktor 1 |

### II. Bioakkumulation

| | | |
|---|---|---|
| Bioakkumulationsfaktor | (BCF) < 500 | Faktor 0,25 |
| Bioakkumulationsfaktor | (BCF) >= 500 - < 2000 | Faktor 0,5 |
| Bioakkumulationsfaktor | (BCF) >= 200 - < 5000 | Faktor 0,75 |
| Bioakkumulationsfaktor | (BCF) > 5000 | Faktor 1,0 |

### III. Akute aquatische Toxizität

| | |
|---|---|
| EC50 > 100 mg/l oder > W | Faktor 0,25 |
| EC50 $\leq$ 100 $\geq$ 10 mg/l | Faktor 0,5 |
| EC50 < 10 $\geq$ 1 mg/l | Faktor 0,75 |
| EC50 < 1 mg/l | Faktor 1 |
| EC50 10-mal niedriger als zuvor | + 0,25 |

### IV. Chronische aquatische Toxizität

| | |
|---|---|
| NOEC/EC10 $\geq$ 10 mg/l | Faktor 0,25 |
| NOEC/EC10 < 10 $\geq$ 1 mg/l | Faktor 0,5 |
| NOEC/EC10 < 1 $\geq$ 0,1 mg/l | Faktor 0,75 |
| NOEC/EC10 < 0,1 $\geq$ 0,01 mg/l | Faktor 1 |
| NOEC/EC10 10-mal niedriger | + 0,25 |

V. <u>Chronische terrestrische Toxizität</u>

| | |
|---|---|
| NOEC/EC10 ≥ 1000 mg/kg | Faktor 0,25 |
| NOEC/EC10 < 1000 ≥ 100 mg/kg | Faktor 0,5 |
| NOEC/EC10 < 100 ≥ 10 mg/kg | Faktor 0,75 |
| NOEC/EC10 < 10 ≥ 1 mg/kg | Faktor 1 |
| NOEC/EC10 10-mal niedriger | + 0,25 |

VI. <u>Sedimenttoxizität</u>

| | |
|---|---|
| NOEC/EC10 ≥ 1000 mg/kg | Faktor 0,25 |
| NOEC/EC10 < 1000 ≥ 100 mg/kg | Faktor 0,5 |
| NOEC/EC10 < 100 ≥ 10 mg/kg | Faktor 0,75 |
| NOEC/EC10 < 10 ≥ 1 mg/kg | Faktor 1 |
| NOEC/EC10 10-mal niedriger | 0,25. |

**7.** Verfahren nach Anspruch 6, wobei das Umweltprofil eine Bewertung von mindestens 0,25 für akute aquatische Toxizität, chronische aquatische Toxizität, chronische terrestrische Toxizität oder Sedimenttoxizität umfasst, wobei eine Bewertung von 0,25 das beste Umweltverhalten darstellt.

**8.** Verfahren nach Anspruch 6 oder 7, wobei das Bestimmen des Umweltprofils auf mindestens einer Datenquelle basiert, die ausgewählt wird aus einer Gruppe bestehend aus experimentellen Daten, ökotoxikologischen Daten, Daten zum Umweltverhalten, Studienberichten, wissenschaftlich fundierter Literatur, Informationen von Regierungsbehörden und qualitativen Struktur-Aktivitäts-Beziehungsmodellen, und/oder

wobei das Bestimmen des Umgebungsprofils ein Evaluieren experimenteller Daten für einen UV-Filter und, wenn keine experimentellen Daten verfügbar sind, dann ein Berechnen oder Schätzen von Daten als Ersatz für die experimentellen Daten umfasst, wobei dann, wenn das Berechnen oder Schätzen von Daten als Ersatz für die experimentellen Daten unmöglich ist, vorzugsweise ein Best-Case-Szenario und ein Worst-Case-Szenarios für den UV-Filter bestimmt wird, und/oder
wobei das Bestimmen des Umweltprofils ein Evaluieren experimenteller Daten für einen UV-Filter und Beurteilen der Zuverlässigkeit der experimentellen Daten umfasst, wobei den experimentellen Daten ein Wert von 1 bis 4 zugeordnet wird und wobei für das Umweltprofil nur Daten mit einer Zuverlässigkeit von 1 oder 2 verwendet werden.

**9.** Verfahren nach einem der Ansprüche 6 bis 8, ferner umfassend ein Einstufen der Mehrzahl von UV-Filtern basierend auf der Umweltgefährdungsgesamtnote.

**Revendications**

**1.** Procédé de formulation d'une composition, comprenant :

la détermination d'un profil environnemental de chacun d'une pluralité de filtres ultraviolets (UV) ;
la détermination d'un score de risque environnemental global de chaque filtre UV, chacun des filtres UV étant sélectionné pour avoir un score de risque environnemental global de 1,5 à 7,0 ;
la préparation de la composition comprenant au moins deux des filtres UV et au moins un autre ingrédient ;
la détermination d'une évaluation écotoxicologique du système de filtres utilisé « a » de la composition ; et
la détermination d'une valeur écotoxicologique (EcoSun Pass value) de la composition,
dans lequel la valeur écotoxicologique va de 0 à 350, dans lequel le score de risque environnemental global est une somme de notes pour six volets environnementaux, dans lequel les volets environnementaux sont choisis dans un groupe constitué par la biodégradation, la bioaccumulation, la toxicité aquatique aiguë, la toxicité aquatique chronique, la toxicité terrestre chronique et
la toxicité sédimentaire, et dans lequel le profil environnemental de chaque filtre UV est basé sur des données de devenir environnemental et des données écotoxicologiques du filtre UV, dans lequel le profil environnemental pour chaque filtre UV comprend une note pour chaque volet environnemental choisi dans un groupe constitué par la biodégradation, la bioaccumulation, la toxicité aquatique aiguë, la toxicité aquatique chronique, la toxicité terrestre chronique et la toxicité sédimentaire, et

dans lequel la détermination de l'évaluation écotoxicologique du système de filtres utilisé « a » de la composition comprend la multiplication du score de risque environnemental global pour chaque filtre UV par sa concentration respective (en poids) dans la composition et l'addition des valeurs résultantes pour chaque filtre UV ;

dans lequel la valeur écotoxicologique (EcoSun Pass value) est représentée par

$$EcoSun\ Pass\ value = a\frac{SPF + UVA - PF}{[UV\ filters]}$$

dans lequel « a » correspond à l'évaluation écotoxicologique du système de filtres utilisé ;

« SPF » correspond à la valeur numérique pour le facteur de protection solaire obtenue à partir d'un simulateur d'écran solaire ou obtenue à partir d'études in vivo ou in vitro ou au moyen du SPF revendiqué, « UVA-PF » est la valeur numérique pour le facteur de protection contre les UVA obtenue à partir d'un simulateur d'écran solaire ou à partir d'études in vivo ou in vitro ou au moyen de l'UVA-PF revendiqué, et « [UV filters] » fait référence à la concentration de filtres UV en pourcentage pondéral, et

dans lequel les notes pour les volets environnementaux sont déterminées comme suit :

I. Biodégradation (selon les critères de l'OCDE)

| | |
|---|---|
| Facilement biodégradable/non persistant | Facteur 0,25 |
| Biodégradable/non persistant | Facteur 0,5 |
| Partiellement biodégradable/potentielleme nt persistant | Facteur 0,75 |
| Difficilement biodégradable/persistant | Facteur 1 |

II. Bioaccumulation

| | | |
|---|---|---|
| Facteur de bioaccumulation (BCF) | < 500 | Facteur 0,25 |
| Facteur de bioaccumulation (BCF) | >= 500 - < 2000 | Facteur 0,5 |
| Facteur de bioaccumulation (BCF) | >= 200 - < 5000 | Facteur 0,75 |
| Facteur de bioaccumulation (BCF) | > 5000 | Facteur 1,0 |

III. Toxicité aquatique aiguë

| | |
|---|---|
| EC50 > 100 mg/l ou > W | Facteur 0,25 |
| EC50 ≤ 100 ≥ 10 mg/l | Facteur 0,5 |
| EC50 < 10 ≥ 1 mg/l | Facteur 0,75 |
| EC50 < 1 mg/l | Facteur 1 |
| EC50 10 fois plus faible que précédemment | + 0,25 |

IV. Toxicité aquatique chronique

| | |
|---|---|
| NOEC/EC10 ≥ I0 mg/l | Facteur 0,25 |
| NOEC/EC10 < 10 ≥ 1 mg/l | Facteur 0,5 |
| NOEC/EC10 < 1 ≥ 0,1 mg/l | Facteur 0,75 |
| NOEC/EC10 < 0,1 ≥ 0,01 mg/l | Facteur 1 |
| NOEC/EC10 10 fois plus faible | + 0,25 |

V. Toxicité terrestre chronique

| | |
|---|---|
| NOEC/EC10 ≥ 1000 mg/kg | Facteur 0,25 |
| NOEC/EC10 < 1000 ≥ 100 mg/kg | Facteur 0,5 |

(suite)

| | |
|---|---|
| NOEC/EC10 < 100 ≥ 10 mg/kg | Facteur 0,75 |
| NOEC/EC10 < 10 ≥ 1 mg/kg | Facteur 1 |
| NOEC/EC10 10 fois plus faible | + 0,25 |

VI. <u>Toxicité sédimentaire</u>

| | |
|---|---|
| NOEC/EC10 ≥ 1000 mg/kg | Facteur 0,25 |
| NOEC/EC10 < 1000 ≥ 100 mg/kg | Facteur 0,5 |
| NOEC/EC10 < 100 ≥ 10 mg/kg | Facteur 0,75 |
| NOEC/EC10 < 10 ≥ 1 mg/kg | Facteur 1 |
| NOEC/EC10 10 fois plus faible | + 0,25. |

**2.** Procédé selon la revendication 1, dans lequel l'au moins un autre ingrédient forme une base d'une composition d'écran solaire, de préférence dans lequel l'au moins un autre ingrédient est choisi dans un groupe constitué par un tensioactif, un agent surgraissant, une huile, un émulsifiant, une cire, un régulateur de consistance, un épaississant, des polymères, une silicone, un siloxane, un stabilisant, un ingrédient actif biogénique, un triglycéride naturel ou synthétique, un acide gras, un alcool gras, un ester d'acide gras, un agent gonflant, un autre agent de protection contre les UV, un antioxydant, un agent hydrotrope, un conservateur, un solubilisant, une huile parfumée, un colorant, un agent inhibant les bactéries, un adjuvant et les mélanges de ceux-ci.

**3.** Procédé selon la revendication 1 ou 2,

dans lequel le profil environnemental comprend une note allant de 0,25 à 1 pour au moins un paramètre parmi la biodégradation et la bioaccumulation, une note de 0,25 représentant le meilleur comportement environnemental et une note de 1,0 représentant le pire comportement environnemental, et/ou
dans lequel le profil environnemental comprend une note d'au moins 0,25 pour au moins un paramètre parmi la toxicité aquatique aiguë, la toxicité aquatique chronique, la toxicité terrestre chronique et la toxicité sédimentaire, une note de 0,25 représentant le meilleur comportement environnemental.

**4.** Procédé selon l'une quelconque des revendications 1 à 3, dans lequel la détermination du profil environnemental est basée sur au moins une source de données choisie dans un groupe constitué par des données expérimentales, des données écotoxicologiques, des données de devenir environnemental, des rapports d'études, la littérature scientifique, des informations émanant d'autorités gouvernementales et des modèles qualitatifs de relations structure-activité, et/ou dans lequel la détermination du profil environnemental comprend l'évaluation de données expérimentales pour un filtre UV, et si aucune donnée expérimentale n'est disponible, alors le calcul ou l'estimation de données pour remplacer les données expérimentales, de préférence dans lequel, si le calcul ou l'estimation de données pour remplacer les données expérimentales est impossible, alors un scénario optimiste et un scénario pessimiste sont déterminés pour le filtre UV, et/ou dans lequel la détermination du profil environnemental comprend l'évaluation de données expérimentales pour un filtre UV et la détermination de la fiabilité des données expérimentales, dans lequel les données expérimentales se voient attribuer une valeur de 1 à 4, et dans lequel seules les données avec une fiabilité de 1 ou 2 sont utilisées pour le profil environnemental.

**5.** Procédé selon l'une quelconque des revendications 1 à 4, comprenant en outre le classement de la pluralité de filtres UV sur la base du score de risque environnemental global.

**6.** Procédé d'utilisation d'un score de risque environnemental global de chacun d'une pluralité de filtres ultraviolets (UV) pour la formulation d'une composition présentant une valeur écotoxicologique (EcoSun Pass value) de 0 à 350, le procédé comprenant :

la détermination d'un profil environnemental de chacun de la pluralité de filtres UV ;
la détermination du score de risque environnemental global de chaque filtre UV ;
la préparation de la composition comprenant au moins deux des filtres UV ;
la détermination d'une évaluation écotoxicologique du système de filtres utilisé « a » de la composition ; et
la détermination d'une valeur écotoxicologique de la composition, dans lequel le profil environnemental de chaque filtre UV est basé sur des données de devenir environnemental et des données écotoxicologiques du filtre UV,

dans lequel le score de risque environnemental global est une somme de notes pour six volets environnementaux, dans lequel les volets environnementaux sont choisis dans un groupe constitué par la biodégradation, la bioaccumulation, la toxicité aquatique aiguë, la toxicité aquatique chronique, la toxicité terrestre chronique et la toxicité sédimentaire,

dans lequel la détermination de l'évaluation écotoxicologique du système de filtres utilisé « a » de la composition comprend la multiplication du score de risque environnemental global pour chaque filtre UV par sa concentration respective (en poids) dans la composition et l'addition des valeurs résultantes pour chaque filtre UV,

dans lequel le profil environnemental pour chaque filtre UV comprend une note pour chaque volet environnemental choisi dans un groupe constitué par la biodégradation, la bioaccumulation, la toxicité aquatique aiguë, la toxicité aquatique chronique, la toxicité terrestre chronique et la toxicité sédimentaire,

dans lequel la valeur EcoSun Pass value est représentée par

$$EcoSun\ Pass\ value = a\frac{SPF + UVA - PF}{[UV\ filters]}$$

dans lequel « a » correspond à l'évaluation écotoxicologique du système de filtres utilisé ;

« SPF » correspond à la valeur numérique pour le facteur de protection solaire obtenue à partir d'un simulateur d'écran solaire ou obtenue à partir d'études in vivo ou in vitro ou au moyen du SPF revendiqué, « UVA-PF » est la valeur numérique pour le facteur de protection contre les UVA obtenue à partir d'un simulateur d'écran solaire ou à partir d'études in vivo ou in vitro ou au moyen de l'UVA-PF revendiqué, et « [UV filters] » fait référence à la concentration de filtres UV en pourcentage pondéral, dans lequel le profil environnemental comprend une note allant de 0,25 à 1 pour la biodégradation ou la bioaccumulation, une note de 0,25 représentant le meilleur comportement environnemental et une note de 1,0 représentant le pire comportement environnemental, et dans lequel les notes pour les volets environnementaux sont déterminées comme suit :

I. <u>Biodégradation (selon les critères de l'OCDE)</u>

| | |
|---|---|
| Facilement biodégradable/non persistant | Facteur 0,25 |
| Biodégradable/non persistant | Facteur 0,5 |
| Partiellement biodégradable/potentiellement persistant | Facteur 0,75 |
| Difficilement biodégradable/persistant | Facteur 1 |

II. <u>Bioaccumulation</u>

| | | |
|---|---|---|
| Facteur de bioaccumulation (BCF) | < 500 | Facteur 0,25 |
| Facteur de bioaccumulation (BCF) | >= 500 - < 2000 | Facteur 0,5 |
| Facteur de bioaccumulation (BCF) | >= 200 - < 5000 | Facteur 0,75 |
| Facteur de bioaccumulation (BCF) | > 5000 | Facteur 1,0 |

III. <u>Toxicité aquatique aiguë</u>

| | |
|---|---|
| EC50 > 100 mg/l ou > W | Facteur 0,25 |
| EC50 $\leq$ 100 $\geq$ 10 mg/l | Facteur 0,5 |
| EC50 < 10 $\geq$ 1 mg/l | Facteur 0,75 |
| EC50 < 1 mg/l | Facteur 1 |
| EC50 10 fois plus faible que précédemment | + 0,25 |

IV. <u>Toxicité aquatique chronique</u>

| | |
|---|---|
| NOEC/EC10 $\geq$ 10 mg/l | Facteur 0,25 |
| NOEC/EC10 < 10 $\geq$ 1 mg/l | Facteur 0,5 |
| NOEC/EC10 < 1 $\geq$ 0,1 mg/l | Facteur 0,75 |

(suite)

NOEC/EC10 < 0,1 ≥ 0,01 mg/l    Facteur 1
NOEC/EC10 10 fois plus faible    + 0,25

V. <u>Toxicité terrestre chronique</u>

NOEC/EC10 ≥ 1000 mg/kg         Facteur 0,25
NOEC/EC10 < 1000 ≥ 100 mg/kg    Facteur 0,5
NOEC/EC10 < 100 ≥ 10 mg/kg      Facteur 0,75
NOEC/EC10 < 10 ≥ 1 mg/kg        Facteur 1
NOEC/EC10 10 fois plus faible   + 0,25

VI. <u>Toxicité sédimentaire</u>

NOEC/EC10 ≥ 1000 mg/kg         Facteur 0,25
NOEC/EC10 < 1000 ≥ 100 mg/kg    Facteur 0,5
NOEC/EC10 < 100 ≥ 10 mg/kg      Facteur 0,75
NOEC/EC10 < 10 ≥ 1 mg/kg        Facteur 1
NOEC/EC10 10 fois plus faible   + 0,25.

**7.** Procédé selon la revendication 6, dans lequel le profil environnemental comprend une note d'au moins 0,25 pour la toxicité aquatique aiguë, la toxicité aquatique chronique, la toxicité terrestre chronique ou la toxicité sédimentaire, une note de 0,25 représentant le meilleur comportement environnemental.

**8.** Procédé selon la revendication 6 ou 7, dans lequel la détermination du profil environnemental est basée sur au moins une source de données choisie dans un groupe constitué par des données expérimentales, des données écotoxicologiques, des données de devenir environnemental, des rapports d'études, la littérature scientifique, des informations émanant d'autorités gouvernementales et des modèles qualitatifs de relations structure-activité, et/ou

dans lequel la détermination du profil environnemental comprend l'évaluation de données expérimentales pour un filtre UV, et si aucune donnée expérimentale n'est disponible, alors le calcul ou l'estimation de données pour remplacer les données expérimentales, de préférence dans lequel, si le calcul ou l'estimation de données pour remplacer les données expérimentales est impossible, alors un scénario optimiste et un scénario pessimiste sont déterminés pour le filtre UV, et/ou
dans lequel la détermination du profil environnemental comprend l'évaluation de données expérimentales pour un filtre UV et la détermination de la fiabilité des données expérimentales, dans lequel les données expérimentales se voient attribuer une valeur de 1 à 4, et dans lequel seules les données avec une fiabilité de 1 ou 2 sont utilisées pour le profil environnemental.

**9.** Procédé selon l'une quelconque des revendications 6 à 8, comprenant en outre le classement de la pluralité de filtres UV sur la base du score de risque environnemental global.

Collect data for an individual component from
available data sources, wherein the data relates to at
least one of a) biodegradation, b) bioaccumulation, c)
acute aquatic toxicity, d) chronic aquatic toxicity, e)
chronic terrestrial toxicity or f) sediment toxicity
105

100

Is data
unavailable
for any of a) – f)?
110

Yes →

Evaluate the available core data of
the individual component
130

No

Assess the reliability of the available data
115

Are the basic/
core data unavailable?
135

Yes →

Determine a worst-case scenario to
fill the data gap
145

No

Develop an expert statement to fill
the data gap
140

Rank each environmental chapter a) – f) for
the individual component to determine the
environmental profile
120

Determine the overall environmental hazard
score of the individual component
125

FIG. 1

34

200

Combine at least one UV filter with at least one other
ingredient, wherein the UV filter has an overall
environmental hazard score of 1.5 to 4.0
<u>205</u>

Determine the ecotoxicological value of the
combination, wherein the value ranges from 1.5 to 37.5
<u>210</u>

FIG. 2

300

```
┌─────────────────────────────────────────────┐
│  Determine the environmental profile of each of a │
│              plurality of UV filters          │
│                                          305  │
└─────────────────────────────────────────────┘
                        │
                        ▼
┌─────────────────────────────────────────────┐
│  Determine an overall environmental hazard score of │
│                 each UV filter                │
│                                          310  │
└─────────────────────────────────────────────┘
                        │
                        ▼
┌─────────────────────────────────────────────┐
│  Prepare a composition containing at least one of the │
│               profiled UV filters             │
│                                          315  │
└─────────────────────────────────────────────┘
                        │
                        ▼
┌─────────────────────────────────────────────┐
│    Determine the ecotoxicological value of the │
│                 composition                   │
│                                          320  │
└─────────────────────────────────────────────┘
```

FIG. 3

**REFERENCES CITED IN THE DESCRIPTION**

**Patent documents cited in the description**

- EP 1093796 A

**Non-patent literature cited in the description**

- *Cosm. Toil.,* 1976, vol. 91, 27
- **K. F. DEPOLO.** A short textbook of cosmetology. 250-251
- **K. F. DEPOLO.** A short textbook of cosmetology. 210-219
- Kosmetische Färbemittel. Farbstoffkommission der Deutschen Forschungsgemeinschaft. Verlag Chemie, 1984, 81-106